# EUROPEAN PATENT APPLICATION

(11) **EP 2 248 840 A1**
(43) Date of publication of application: **10.11.2010**
(21) Application number: 09710447.5
(22) Date of filing: 13.02.2009
(51) Int. Cl.: C08G 61/12, C07D 495/04, H01L 29/786, H01L 51/05, H01L 51/30

(54) **CONDENSED POLYCYCLIC COMPOUND, CONDENSED POLYCYCLIC POLYMER AND ORGANIC THIN FILM CONTAINING THE COMPOUND OR THE POLYMER**

(30) Priority: 13.02.2008 JP 2008032245
(71) Applicant: Osaka University, Suita-shi Osaka 565-0871 (JP); Sumitomo Chemical Company, Limited, Tokyo 104-8260 (JP)
(72) Inventor: MIURA, Masahiro, Suita-shi Osaka 565-0871 (JP); SATOH, Tetsuya, Suita-shi Osaka 565-0871 (JP); TSURUGI, Hayato, Suita-shi Osaka 565-0871 (JP); KUMAGAI, Jun, Suita-shi Osaka 565-0871 (JP); UEDA, Masato, Tsukuba-shi Ibaraki 305-0046 (JP)
(74) Representative: Duckworth, Timothy John
(86) International application number: PCT/JP2009/052429
(87) International publication number: WO 2009/102031

(57) **Abstract**

An object of the present invention is to provide a polycyclic fusedring polymer which has a structure capable of showing high charge transport properties and also is excellent in solubility into a solvent. The polycyclic fusedring polymer according to the present invention includes: a plurality of one of a first structural unit represented by the following formula (1) and a second structural unit represented by the following formula (2); or the first structural unit and the second structural unit in combination: 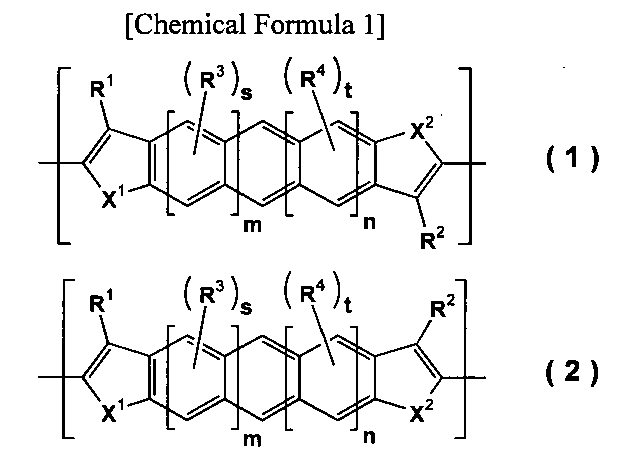 wherein X¹ and X² represent an oxygen atom, a sulfur atom or a selenium atom, and R¹ and R² represent a halogen atom, an unsaturated alkyl group, an alkyl group, a non-alkyl group containing an alkyl group, an aryl group, or a monovalent heterocyclic group; R³ and R⁴ represent a monovalent group, and when a plurality of R³ and R⁴ exist, these may be the same or different; and m, n, s and t are an integer of 0 to 2.

## Description

### Technical Field

The present invention relates to a polycyclic fusedring compound, a polycyclic fusedring polymer, an organic film containing them, and an organic film device and an organic film transistor each comprising the organic film.

### Background Art

A thin film containing an organic material having charge (electron or hole) transport properties is expected to be applied to organic film devices such as an organic film transistor, an organic solar cell and a photosensor, and the development of an organic p-type semiconductor material (which shows hole transport properties) and an organic n-type semiconductor material (which shows electron transport properties) which can form such a thin film are variously studied.

As for the organic p-type semiconductor material, a compound having a thiophene ring such as oligothiophene and polythiophene is expected to show high hole transport properties because the compound can be in a stable radical cation state. The oligothiophene having a long chain length particularly has a long conjugation length, and accordingly is anticipated to have higher hole transport properties. However, among straight molecules, sufficient interaction between molecules is not obtained because the regularity of molecular arrays is poor in a solid state, and as high hole transport properties as expected have not yet been obtained.

In addition, in order to enhance the planarity of the molecular structure to obtain the high charge transport properties, polythiophene having a crosslinked structure, a ladder type fluorene and the like are studied (Patent Document 1).
Patent Document 1: U.S. Patent No. 2004/186266

### Disclosure of the Invention

### Problems to be Solved by the Invention

An advantage of the organic material having charge transport properties as described above is that a thin film can easily be formed by dissolving the material in a solvent or the like and applying it. However, in order to obtain an organic film device having high characteristics, the organic material needs to have high charge transport properties in a state of being formed into an organic film, but the organic material structurally having high charge transport properties as described above often cannot provide high charge transport properties when the material is formed into the organic film. This is considered to be partly because a conventional organic material having a structure of high charge transport properties has low solubility in a solvent and thus is resistant to formation of a uniform film when the material is formed into an organic film.

Then, the present invention is designed with respect to such circumstances, and is directed at providing a polycyclic fusedring polymer and a polycyclic fused ring compound which have a structure capable of showing high charge transport properties and also have excellent solubility in the solvent. The present invention is also directed at providing a polycyclic fusedring compound for obtaining a polycyclic fusedring polymer, an organic film containing the polycyclic fusedring polymer or the polycyclic fusedring compound, and an organic film device comprising the organic film such as an organic film transistor.

### Means for Solving the Problem

In order to achieve the above described object, the polycyclic fusedring polymer according to the present invention includes: two or more first structural units represented by the following formula (1) and/or two or more second structural units represented by the following formula (2); or the first structural unit and the second structural unit in combination: 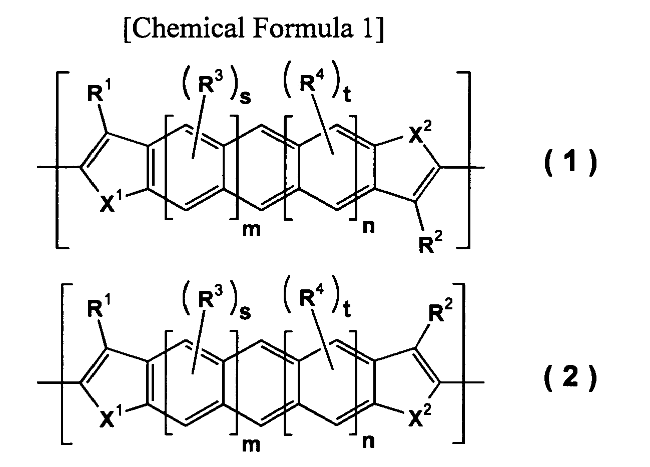 wherein X¹ and X² each independently represent an oxygen atom, a sulfur atom or a selenium atom and R¹ and R² each independently represent a halogen atom, an unsaturated alkyl group, a straight, branched or cyclic alkyl group having 8 to 20 carbon atoms, a non-alkyl group containing a straight, branched or cyclic alkyl group, an aryl group that has 6 to 60 carbon atoms and may have a substituent or a monovalent heterocyclic group that has 4 to 60 carbon atoms and may have a substituent; R³ and R⁴ each independently represent a monovalent group that may have a substituent, and when there are a plurality of R³s and a plurality of R⁴s, the R³s may be the same as or different from each other and the R⁴s may be the same as or different from each other; and m, n, s and t are each independently an integer of 0 to 2.

Because such a polycyclic fusedring polymer of the present invention has a high planarity of π-conjugation plane by the fusion of a plurality of rings between themselves, the polycyclic fusedring polymer has a structure capable of showing the high charge transport properties (hole transport properties). Such a polycyclic fusedring polymer also has the high solubility to the solvent because of having predetermined substituents of R¹ and R². Accordingly, the polycyclic fusedring polymer of the present invention can adequately form a uniform thin film by application, and can sufficiently provide the high charge transport properties originating in the structure.

In the above described polycyclic fusedring polymer of the present invention, it is preferable if the above described X¹ and X² are sulfur atoms. When having such a structure, the polycyclic fusedring polymer can provide higher charge transport properties.

In addition, it is preferable that the polycyclic fusedring polymer of the present invention have further a structural unit represented by the following formula (3), because it becomes possible to obtain higher charge transport properties and the solubility to the solvent: 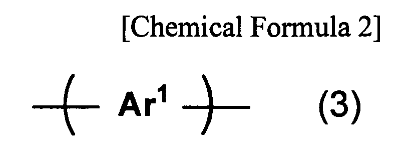 wherein Ar¹ represents a divalent aromatic hydrocarbon group that may have a substituent, or a divalent heterocyclic group that may have a substituent.

Among them, in the structural unit represented by the above formula (3), the group represented by Ar¹ is further preferably a group represented by the following formula (4) because of providing excellent charge transport properties. In addition, in the following formula (4), a group represented by Z¹ is particularly preferably a group represented by the following formula (ii): 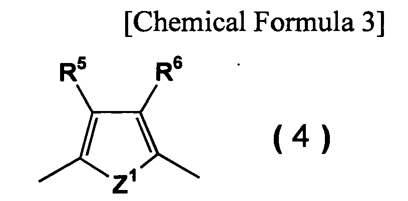 wherein R⁵ and R⁶ each independently represent a hydrogen atom, a halogen atom or a monovalent group and R⁵ and R⁶ may be bonded together to form a ring; and Z³ represents any one of groups represented by the following formulae (i), (ii), (iii), (iv), (v), (vi), (vii), (viii) and (ix) (which will be referred to as "(i) to (ix)", hereinafter): 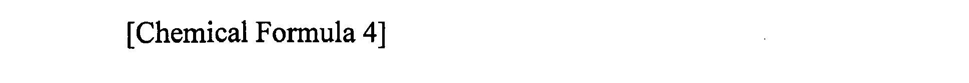 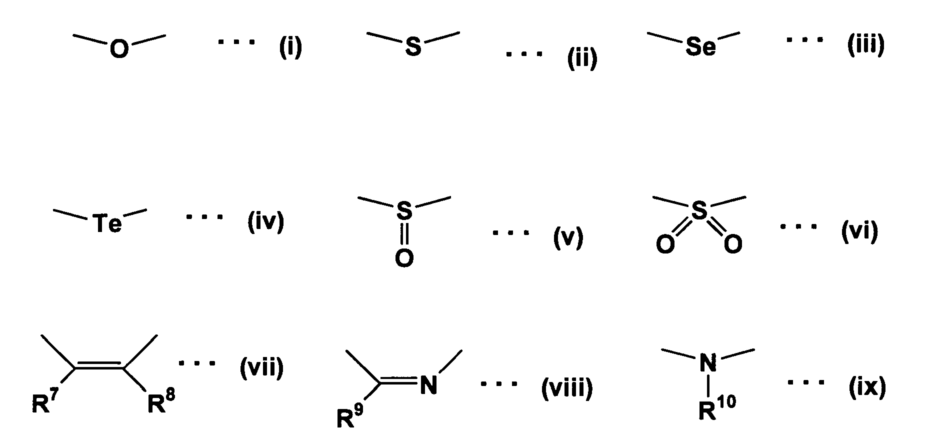 wherein R⁷, R⁸, R⁹ and R¹⁰ each independently represent a hydrogen atom, a halogen atom or a monovalent group, and R⁷ and R⁸ may be bonded together to form a ring; and the left side and right side of the group represented by formula (viii) may be interchanged.

The polycyclic fusedring compound of the present invention is a compound suitable for providing the polycyclic fusedring polymer of the present invention, and is represented by the following formula (5) or the following formula (6): 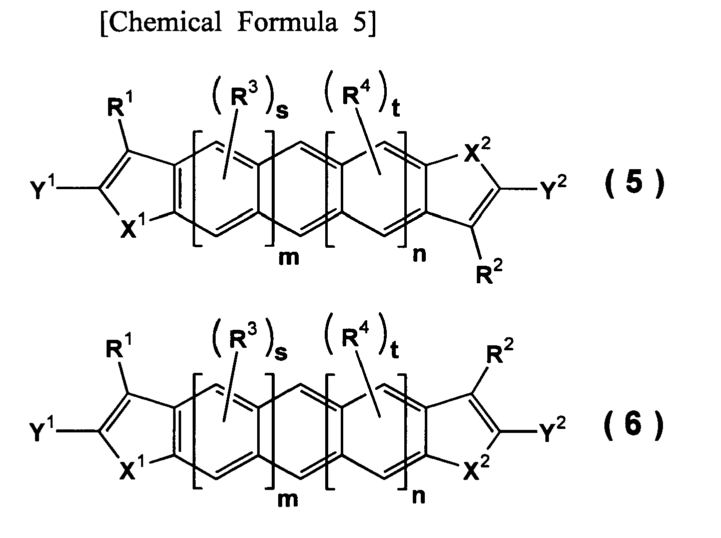 wherein X¹ and X² each independently represent an oxygen atom, a sulfur atom, or a selenium atom, R¹ and R² each independently represent a halogen atom, an unsaturated alkyl group, a straight, branched or cyclic alkyl group having 8 to 20 carbon atoms, a non-alkyl group containing a straight, branched or cyclic alkyl group, an aryl group that has 6 to 60 carbon atoms and may have a substituent, or a monovalent heterocyclic group that has 4 to 60 carbon atoms and may have a substituent; R³ and R⁴ each independently represent a monovalent group that may have a substituent, and when there are a plurality of R³ and a plurality of R⁴s, the R³s may be the same as or different from each other and the R⁴s may be the same as or different from each other; Y¹ and Y² each independently represent a halogen atom, a carboxyl group, an alkoxycarbonyl group, an alkyl sulfonate group, an aryl sulfonate group, an aryl alkyl sulfonate group, an alkyl stannyl group, an aryl stannyl group, an aryl alkyl stannyl group, a boric ester residue, a sulfonium methyl group, a phosphonium methyl group, a methyl phosphonate group, a monohalogenated methyl group, a boric acid residue, a formyl group or a vinyl group; and m, n, s and t are each independently an integer of 0 to 2.

It is also preferable that the polycyclic fusedring compound of the present invention be a compound represented by the following formula (7) or the following formula (8): 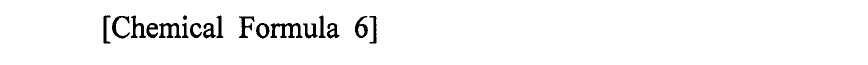 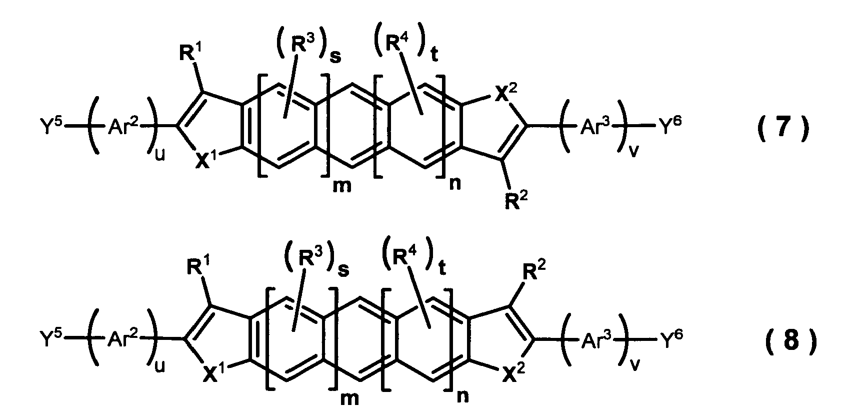 wherein X¹ and X² each independently represent an oxygen atom, a sulfur atom, or a selenium atom, R¹ and R² each independently represent a straight, branched or cyclic alkyl group having 8 to 20 carbon atoms, a non-alkyl group containing a straight, branched or cyclic alkyl group, an aryl group that has 6 to 60 carbon atoms and may have a substituent, or a monovalent heterocyclic group that has 4 to 60 carbon atoms and may have a substituent; R³ and R⁴ each independently represent a monovalent group that may have a substituent, and when there are a plurality of R³s and a plurality of R⁴s, the R³s may be the same as or different from each other and the R⁴s may be the same as or different from each other; Y⁵ and Y⁶ each independently represent a hydrogen atom, a halogen atom, an alkyl group having 1 to 20 carbon atoms, an aryl group that has 6 to 60 carbon atoms and may have a substituent, a monovalent heterocyclic group that has 4 to 60 carbon atoms and may have a substituent, a carboxyl group, an alkoxycarbonyl group, an alkyl sulfonate group, an aryl sulfonate group, an aryl alkyl sulfonate group, an alkyl stannyl group, an aryl stannyl group, an aryl alkyl stannyl group, a boric ester residue, a sulfonium methyl group, a phosphonium methyl group, a methyl phosphonate group, a monohalogenated methyl group, a boric acid residue, a formyl group, or a vinyl group; Ar² and Ar³ each independently represent a divalent aromatic hydrocarbon group that may have a substituent, or a divalent heterocyclic group that may have a substituent; and m, n, s and t are each independently an integer of 0 to 2, and u and v are each independently 0 or 1 wherein u + v is a number of 1 or more.

The above described polycyclic fusedring compounds can easily form the polycyclic fusedring polymers of the present invention by polymerizing the compounds when Y¹, Y², Y⁵ and Y⁶ of the compounds are polymerization-active groups, and accordingly are extremely useful as a raw compound of the polycyclic fusedring polymers of the present invention. In addition, the polycyclic fusedring compound represented by the above formula (7) or (8) is particularly useful by itself as a material having charge transport properties because of having a structure of high charge transport properties and excellent solubility in a solvent.

In such a polycyclic fusedring compound of the present invention, the X¹ and the X² are more preferably a sulfur atom. Thereby, the charge transport properties of the polycyclic fusedring polymer become more adequate.

The present invention also provides an organic film containing the polycyclic fusedring polymer or the polycyclic fused ring compound of the present invention. Since such an organic film is a substantially uniform film because of being obtained with the use of the polycyclic fusedring polymer or the polycyclic fusedring compound of the present invention, the organic film can sufficiently show high charge transport properties which the polycyclic fusedring polymer or the polycyclic fusedring compound originally has.

Furthermore, the present invention provides an organic film device comprising the organic film of the present invention, and particularly provides an organic film transistor. These organic film device and organic film transistor can have excellent characteristics as a device because of comprising the organic film having high charge transport properties.

### Effect of the Invention

The present invention can provide a polycyclic fusedring polymer and a polycyclic fusedring compound which have a structure capable of showing high charge transport properties and also have excellent solubility in a solvent. The present invention also can provide a polycyclic fusedring compound for obtaining a polycyclic fusedring polymer, an organic film containing the polycyclic fusedring polymer or the polycyclic fusedring compound, and an organic film device comprising the organic film such as an organic film transistor.

### Brief Description of the Drawings

Figure 1 is a schematic sectional view of an organic film transistor according to a first embodiment;
Figure 2 is a schematic sectional view of an organic film transistor according to a second embodiment;
Figure 3 is a schematic sectional view of an organic film transistor according to a third embodiment;
Figure 4 is a schematic sectional view of an organic film transistor according to a fourth embodiment;
Figure 5 is a schematic sectional view of an organic film transistor according to a fifth embodiment;
Figure 6 is a schematic sectional view of an organic film transistor according to a sixth embodiment;
Figure 7 is a schematic sectional view of an organic film transistor according to a seventh embodiment;
Figure 8 is a schematic sectional view of a solar cell according to an embodiment;
Figure 9 is a schematic sectional view of a photosensor according to a first embodiment;
Figure 10 is a schematic sectional view of a photosensor according to a second embodiment; and
Figure 11 is a schematic sectional view of a photosensor according to a third embodiment.

### Description of Symbols

1 ...substrate, 2 ... active layer, 2a ... active layer, 3 ... insulation layer, 4 ... gate electrode, 5 ... source electrode, 6 ... drain electrode, 7a ... first electrode, 7b ... second electrode, 8 ... electric-charge-generating layer, 100 ... organic film transistor according to first embodiment, 110 ...organic film transistor according to second embodiment, 120 ... organic film transistor according to third embodiment, 130 ...organic film transistor according to fourth embodiment, 140 ... organic film transistor according to fifth embodiment, 150 ... organic film transistor according to sixth embodiment, 160 ... organic film transistor according to seventh embodiment, 200 ... solar cell according to embodiment, 300 ... photosensor according to first embodiment, 310 ... photosensor according to second embodiment, and 320 ... photosensor according to third embodiment

### Best Modes for Carrying Out the Invention

Preferred embodiments according to the present invention will be described in detail below with reference to the drawings as needed. Incidentally, in the drawings, the same reference characters shall be put on the same elements, and overlapping descriptions are omitted. A positional relation such as up, down, left and right may be based on the positional relation as is illustrated in the drawings, unless otherwise specifically indicated. Furthermore, a dimensional ratio in the drawings is not limited to the ratio shown in the drawings.

### [Polycyclic fusedring polymer]

The polycyclic fusedring polymer according to preferred embodiments has a plurality of at least one of first structural units represented by the above formula (1) and second structural units represented by the above formula (2), or has the first structural unit and the second structural unit in combination. Specifically, the polycyclic fusedring polymer of the present embodiment includes a polymer having a plurality of the first structural units, a polymer having a plurality of the second structural units, and a polymer containing at least each one of the first and second structural units. Here, the "structural unit" of the polycyclic fusedring polymer means a structural unit constituting the main chain of the polymer. The "polymer" means a polymer having at least two of such "structural units", and includes both of compounds which are usually classified into an oligomer or a polymer. It is preferable if the polycyclic fusedring polymer has 30 mol% or more of the first structural unit and/or the second structural unit, and it is more preferable if the polymer has 50 to 95 mol% of the structural unit.

The preferable structure of the structural unit represented by the above formulae (1) and (2) will be described below. Firstly, X¹ and X² in the formulae (1) and (2) each independently represent an oxygen atom, a sulfur atom or a selenium atom, and it is preferable if X¹ and X² are a sulfur atom. It is preferable if X¹ and X² are the same atom from the viewpoint of facilitating the production of the polycyclic fusedring polymer, and it is further preferable if both of them are a sulfur atom, because particularly high charge transport properties can be obtained.

From the viewpoint of obtaining high solubility into a solvent, R¹ and R² preferably are each independently a straight, branched or cyclic alkyl group having 8 to 20 carbon atoms, and a non-alkyl group containing a straight, branched or cyclic alkyl group, and more preferably are the straight, branched or cyclic alkyl group having 8 to 20 carbon atoms, and particularly preferably are the straight alkyl group having 8 to 20 carbon atoms. However, from the viewpoint of the ease of production, it is more preferable if R¹ and R² are the same group.

The alkyl group represented by R¹ and R² includes an octyl group, a nonyl group, a decyl group, an undecyl group, a dodecyl group, a tridecyl group, a tetradecyl group and a pentadecyl group. In addition, the non-alkyl group containing an alkyl group means a group containing an alkyl group and an atom other than a carbon atom, and a group containing an alkyl group and an unsaturated bond; and includes an alkoxy group, an alkyl-alkenyl group, an alkyl-alkynyl group, an alkylamino group and an alkoxycarbonyl group. These alkyl groups and the alkyl groups contained in the non-alkyl group are more preferably an alkyl group having 8 to 16 carbon atoms, and are further preferably an alkyl group having 8 to 12 carbon atoms.

Examples of the aryl group having 6 to 60 carbon atoms can include a phenyl group, a phenyl group having an alkoxy group having 1 to 12 carbon atoms, a phenyl group having an alkyl group having 1 to 12 carbon atoms, 1-naphthyl group and 2-naphthyl group. Among them, an aryl group having 6 to 20 carbon atoms is preferable, and the phenyl group having the alkoxy group having 1 to 12 carbon atoms and the phenyl group having the alkyl group having 1 to 12 carbon atoms are further preferable.

A monovalent heterocyclic group having 3 to 60 carbon atoms includes a thienyl group, a thienyl group having an alkyl group having 1 to 12 carbon atoms, a pyrrolyl group, a furyl group, a pyridyl group and a pyridyl group having an alkyl group having 1 to 12 carbon atoms. Among them, a monovalent heterocyclic group having 3 to 20 carbon atoms is preferable, and the thienyl group, the thienyl group having the alkyl group having 1 to 12 carbon atoms, the pyridyl group and the pyridyl group having the alkyl group having 1 to 12 carbon atoms are more preferable. In the above description, the monovalent heterocyclic group shall mean a group in which at least one atom constituting a ring in an organic group having a cyclic structure is a heteroatom.

In addition, it is preferable that s and t be 1 or 2, from the viewpoint of enhancing the solubility into a solvent. The monovalent group represented by R³ and R⁴ includes an alkyl group, an alkoxy group, a fluoroalkyl group, a fluoroalkoxy group, an aryl group, an arylamino group and a monovalent heterocyclic group. Among them, the alkyl group, the alkoxy group, the fluoroalkyl group, the fluoroalkoxy group, the aryl group or the arylamino group is preferable, and the alkyl group or the aryl group is further preferable. When any of s, t, m and n is 2, a plurality of R³ and R⁴ result in being contained in the molecule, and in that case, the plurality of R³ and R⁴ may be the same or different. However, it is preferable that in combinations of R³ and R³, R⁴ and R⁴, or R³ and R⁴, the groups be the same group, from the viewpoint of easily producing the polycyclic fusedring polymer.

In the above formula (1) or the above formula (2), m and n are each independently an integer of 0 to 2, but m + n is preferably a combination of 0, 1, 2 or 4, and m + n is particularly preferably 0, from the viewpoint of enhancing electric-charge mobility of the polycyclic fusedring polymer.

The polycyclic fusedring polymer of preferred embodiments has preferably a structural unit represented by the above formula (3) in addition to the first structural unit represented by the above formula (1) and/or the second structural unit represented by the above formula (2). By appropriately containing the third structural unit represented by the above formula (3), the polycyclic fusedring polymer can widen the range capable of adjusting mechanical, thermal or electronic characteristics to a preferable degree in addition to the solubility into a solvent.

In this case, in the polycyclic fusedring polymer, the content ratio of the third structural unit to the first structural unit and/or the second structural unit is preferably a ratio of 10 to 1,000 mol of the former with respect to 100 mol of the latter, more preferably is 25 to 400 mol of the former with respect to 100 mol of the latter, and further preferably is 50 to 200 mol of the former with respect to 100 mol of the latter. When satisfying these conditions, the polycyclic fusedring polymer can obtain high electric-charge mobility and also can easily enhance each of the above described characteristics.

In the above formula (3), Ar¹ is a divalent aromatic hydrocarbon group which may have a substituent or a divalent heterocyclic group which may have a substituent. Here, the divalent aromatic hydrocarbon group means an atom group which is left after 2 pieces of hydrogen atoms have been removed from a benzene ring or a polycyclic fusedring; and preferably has 6 to 60 carbon atoms, and more preferably has 6 to 20 carbon atoms. The polycyclic fusedring includes, for instance, a naphthalene ring, an anthracene ring, a tetracene ring, a pentacene ring, a pyrene ring, a perylene ring and a fluorene ring. The divalent aromatic hydrocarbon group or the divalent heterocyclic group is particularly preferably a group formed of an atom group which is left after 2 pieces of hydrogen atoms have been removed from a benzene ring, a pentacene ring, a pyrene ring or a fluorene ring. These divalent aromatic hydrocarbon groups may have a substituent. In this case, the number of carbon atoms in the substituent shall not be included in the number of carbon atoms in the above described preferable divalent aromatic hydrocarbon group. The substituent includes a halogen atom, a saturated or unsaturated hydrocarbon group, an aryl group, an alkoxy group, an aryloxy group, a monovalent heterocyclic group, an amino group, a nitro group and a cyano group.

In addition, the divalent heterocyclic group means an atom group which is left after 2 pieces of hydrogen atoms have been removed from a heterocyclic compound; and preferably has 3 to 60 carbon atoms and more preferably has 3 to 20 carbon atoms. Here, the heterocyclic compound means a compound in which atoms constituting a ring are not only a carbon atom but also include a heteroatom such as an oxygen atom, a sulfur atom, a nitrogen atom, a phosphorus atom, a boron atom and a silicon atom in the ring, among organic compounds having a ring structure. The heterocyclic compound includes thiophene, thienothiophene, dithienothiophene, a compound in which 4 or 5 pieces of thiophene rings are ring-fused, pyrrole, pyridine, pyrimidine, pyrazine and triazine; and thiophene, thienothiophene and dithienothiophene are preferable.

The divalent heterocyclic group may also have a substituent, but the number of carbon atoms of the substituent is not included in the number of carbon atoms of the above described divalent heterocyclic group. The substituent includes a halogen atom, a saturated or unsaturated hydrocarbon group, an aryl group, an alkoxy group, an aryloxy group, a monovalent heterocyclic group, an amino group, a nitro group and a cyano group.

A group represented by the above formula (4) is particularly preferable for a group represented by Ar¹ in the third structural unit represented by the above formula (3). When containing such a group in the structural unit, the polycyclic fusedring polymer can easily obtain more excellent solubility in a solvent and charge transport properties.

Any of groups represented by the above formulae (i), (ii), (iii), (vii), (viii) and (ix) is preferable for Z¹ in the above formula (4), any of groups represented by the above formulae (ii) and (vii) is more preferable, and the group represented by the above formula (ii) is particularly preferable. When Z¹ is a group represented by the above formula (i), (ii) or (ix), the group represented by the above formula (4) has a structure of a thiophene ring, a furan ring or a pyrrole ring respectively, and these rings, particularly the thiophene ring, show preferable electrical properties and accordingly can show various electrical characteristics.

In addition, R⁵ to R¹⁰ are each independently a hydrogen atom, a halogen atom or a monovalent group, but when R⁵ and R⁶, or R⁷ and R⁸, are a monovalent group, these may be bonded to each other to form a ring. A group having a straight or branched group having a low-molecular chain and a monovalent cyclic group are preferable for the monovalent group represented by R⁵ to R¹⁰. In the above description, the monovalent cyclic group may be any one of a monocycle and a fusedring, any one of a hydrocarbon ring and a heterocycle, any one of a saturated ring and an unsaturated ring, and may further have a substituent. These monovalent groups may be an electron-donating group and may be an electron-attracting group.

Among them, a group having the above described low-molecular chain having 1 to 20 carbon atoms and the above described cyclic group in which the number of atoms constituting the cyclic group is 3 to 60 are preferable for the monovalent group of R⁵ to R¹⁰. Specifically, more preferable monovalent groups are a saturated or an unsaturated hydrocarbon group, a hydroxy group, an alkoxy group, an alkanoyloxy group, an amino group, an oxyamino group, an alkylamino group, a dialkylamino group, an alkanoyl amino group, a cyano group, a nitro group, a sulfonic group, an alkyl group which is substituted with one or more halogen atoms, an alkoxy sulfonyl group (though its alkyl group may be substituted with one or more halogen atoms), an alkyl sulfonyl group (though the alkyl group may be substituted with one or more halogen atoms), a sulfamoyl group, an alkyl sulfamoyl group, a carboxyl group, a carbamoyl group, an alkyl carbamoyl group, an alkanoyl group and an alkoxycarbonyl group.

A methyl group, an ethyl group, an n-propyl group, an isopropyl group, an n-butyl group, an isobutyl group, a sec-butyl group or a tert-butyl group is preferable for the saturated hydrocarbon group (alkyl group) in the above described monovalent groups. In addition, similar groups to the above described alkyl groups are preferable for the alkyl group in the group containing the alkyl group in its structure (such as the alkoxy group, the alkylamino group and the alkoxycarbonyl group), in the above described monovalent groups.

The unsaturated hydrocarbon group includes a vinyl group, 1-propenyl group, an allyl group, a propargyl group, an isopropenyl group, 1-butenyl group and 2-butenyl group; and the vinyl group is preferable.

The alkanoyl group includes a formyl group, an acetyl group, a propionyl group, an isobutyryl group, a valeryl group and an isovaleryl group. An alkanoyl group in the group containing the alkanoyl group in its structure (such as the alkanoyloxy group and the alkanoyl amino group) is similar to the above. Here, the alkanoyl group having 1 carbon atom means a formyl group. Preferable alkanoyl groups include the formyl group and the acetyl group. Furthermore, the halogen atoms include a fluorine atom, a chlorine atom, a bromine atom and an iodine atom.

Structures represented by the following formulae (9) to (19) are preferable for the polycyclic fusedring polymer of the present embodiment having the above described structure. The reference characters in the following formulae (9) to (19) are synonymous with the above description. In addition, Ar¹¹ and k' are synonymous with Ar¹ and k, respectively, and Ar¹¹ may be the same group as or a different group from the group of Ar¹. Furthermore, when a plurality of the same reference characters results in existing in the following structure, these may be each the same or different.

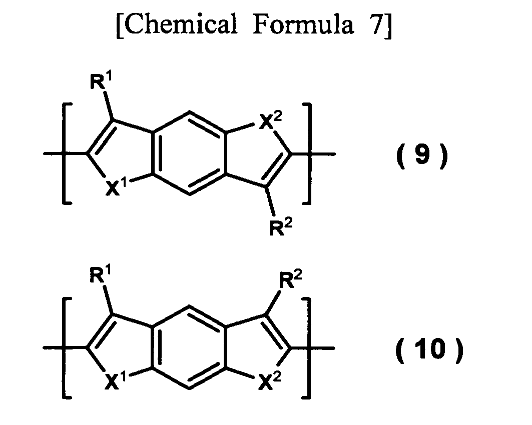

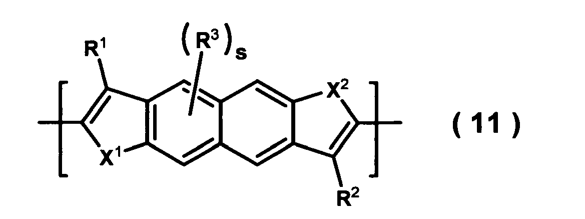

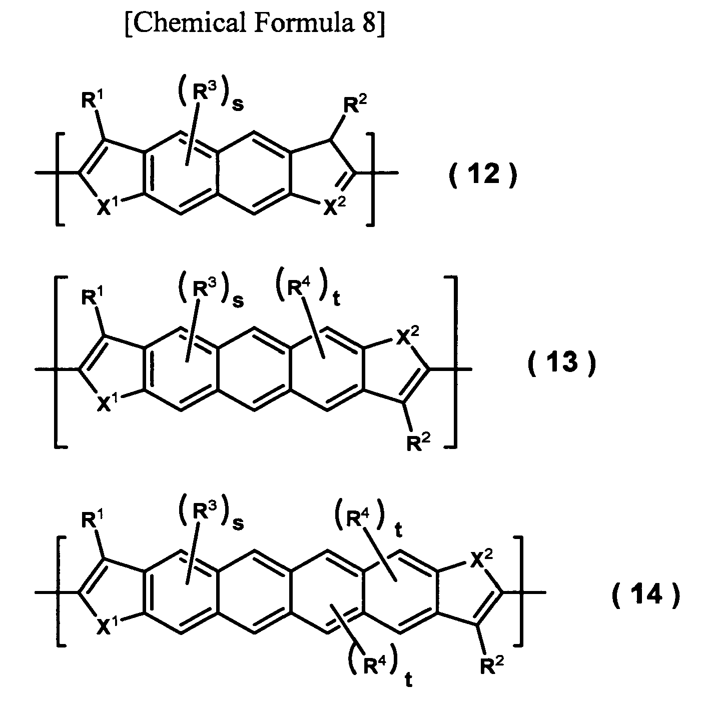

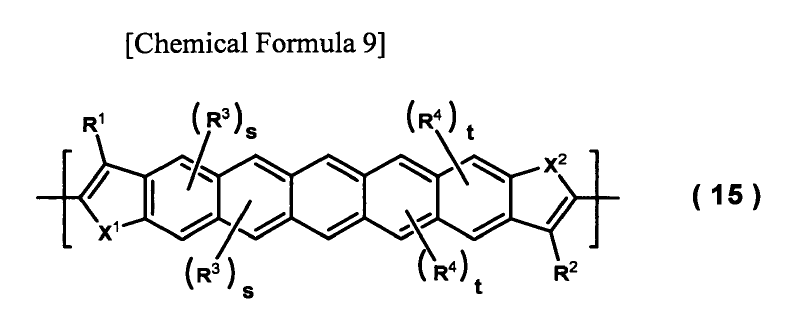

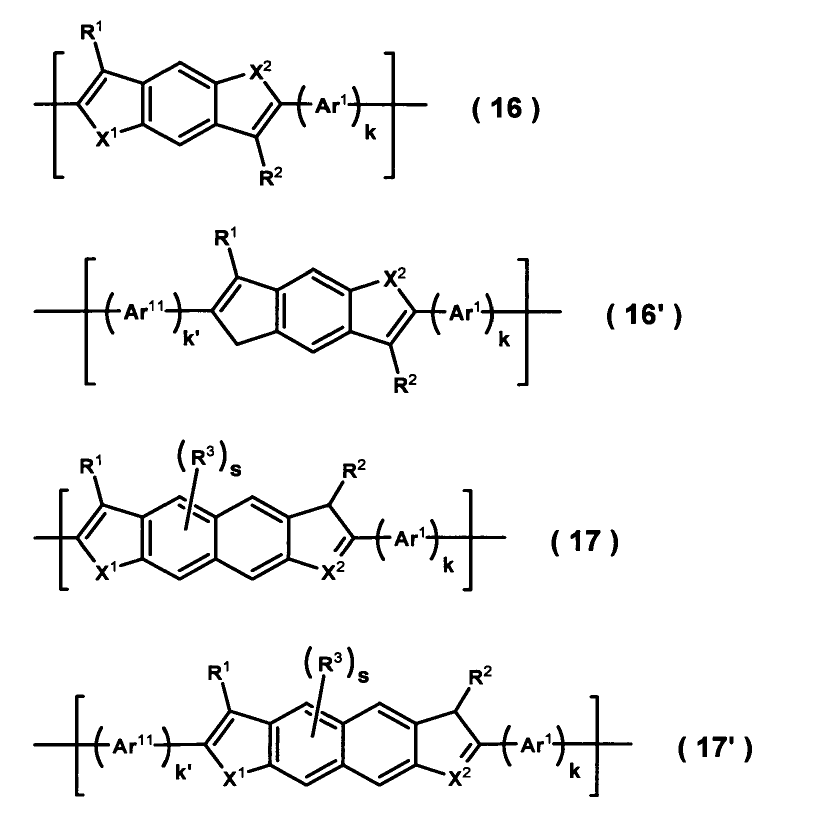

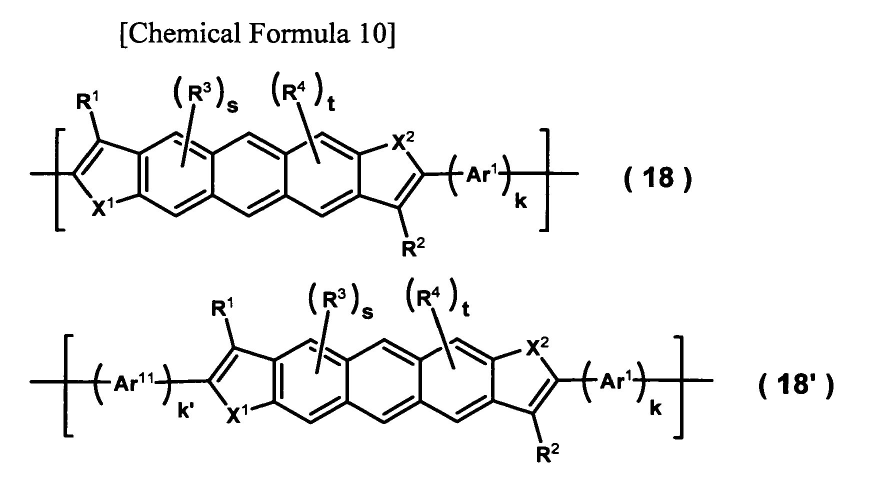

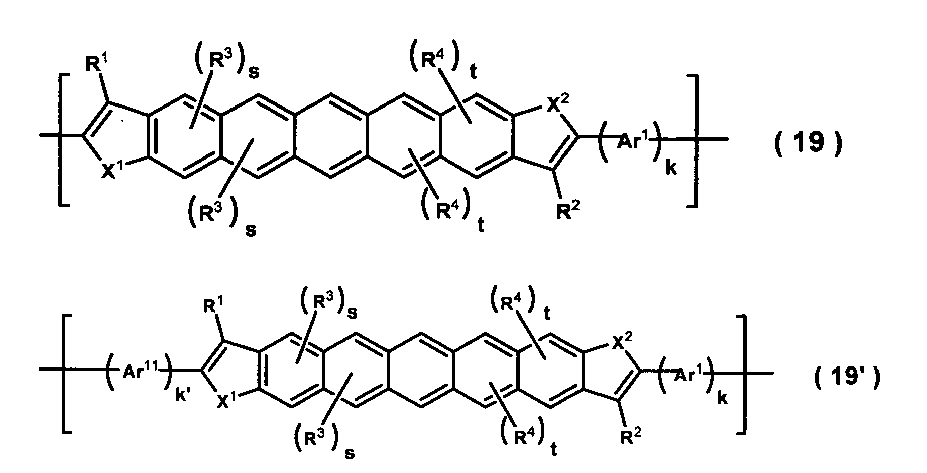

A terminal group of the polycyclic fusedring polymer includes a hydrogen atom, a fluorine atom, an alkyl group, an alkoxy group, an acyl group, an aminoketo group, an aryl group, a monovalent heterocyclic group (in which one part or all of hydrogen atoms bonded to these groups may be substituted with fluorine atoms), a group having an α-fluoroketone structure, and other electron-donating groups and electron-attracting groups. Among them, the alkyl group, the alkoxy group, the aryl group and the monovalent heterocyclic group are preferable. It is also preferable for the terminal group to have a continuous conjugated bond with a conjugate structure of the main chain. Such terminal groups include an aryl group bonded to the main chain through a carbon-carbon bond, and a monovalent heterocyclic group.

Furthermore, the terminal group of the polycyclic fusedring polymer includes also a polymerization-active group. When the polymer has the polymerization-active group for the terminal group, the polycyclic fusedring polymer can also be used as a precursor for obtaining the polycyclic fusedring polymer with a high molecular weight. When the polymer is used as such a precursor, the polycyclic fusedring polymer has preferably two polymerization-active groups in the molecule.

Examples of the polymerization-active group include a halogen atom, a carboxyl group, an alkoxy carbonyl group, an alkyl sulfonate group, an aryl sulfonate group, an aryl alkyl sulfonate group, an alkyl stannyl group, an aryl stannyl group, an aryl alkyl stannyl group, a boric ester residue, a sulfonium methyl group, a phosphonium methyl group, a methyl phosphonate group, a monohalogenated methyl group, a boric acid residue (a group represented by -B(OH)₂), a formyl group and a vinyl group. Among them, the halogen atom, the alkyl stannyl group and the boric ester group are preferable. Here, the boric ester residue is a monovalent group having a structure in which one bond of a boron atom in a boric acid ester is substituted with a bond for substitution, and includes the groups represented by the following formulae (100a) to (100d).

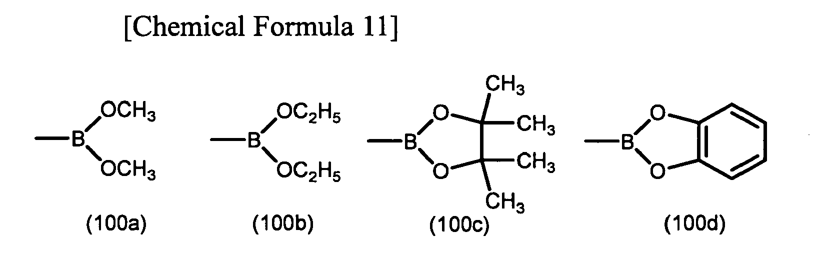

When the polycyclic fusedring polymer is used as an organic film, the polymerization-active group may also be protected with a stable group, because if the polymerization-active group remains as the terminal group as it is, the characteristics and durability of the organic film device which has been formed may be lowered.

The polycyclic fusedring polymer having a structure represented by the following formulae (20) to (30) is particularly preferable, because of being capable of coping with both higher electric-charge mobility and excellent solubility in the solvent.

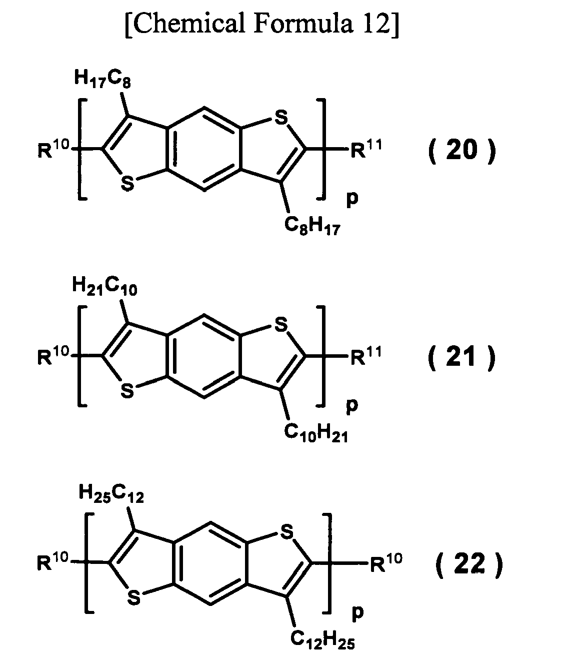

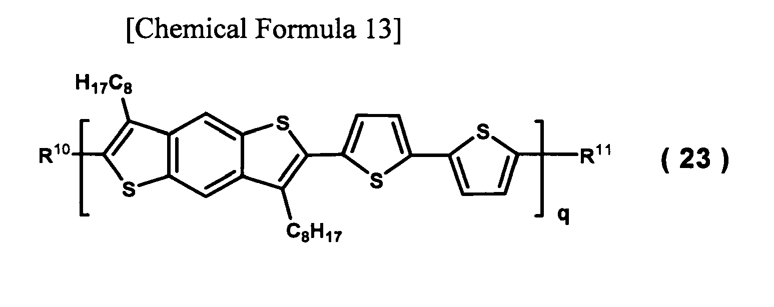

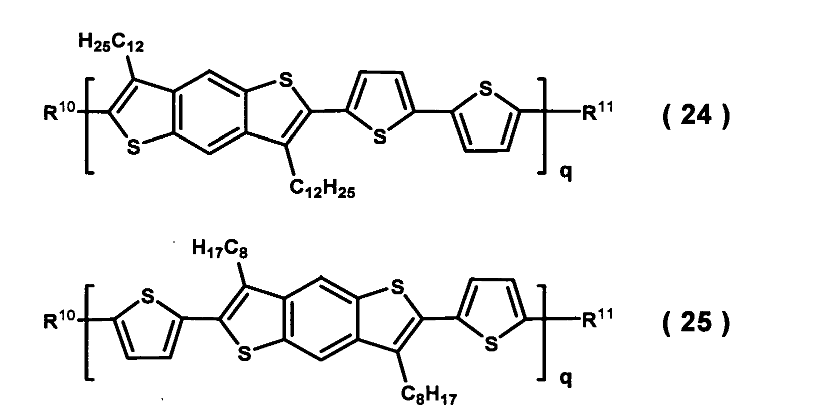

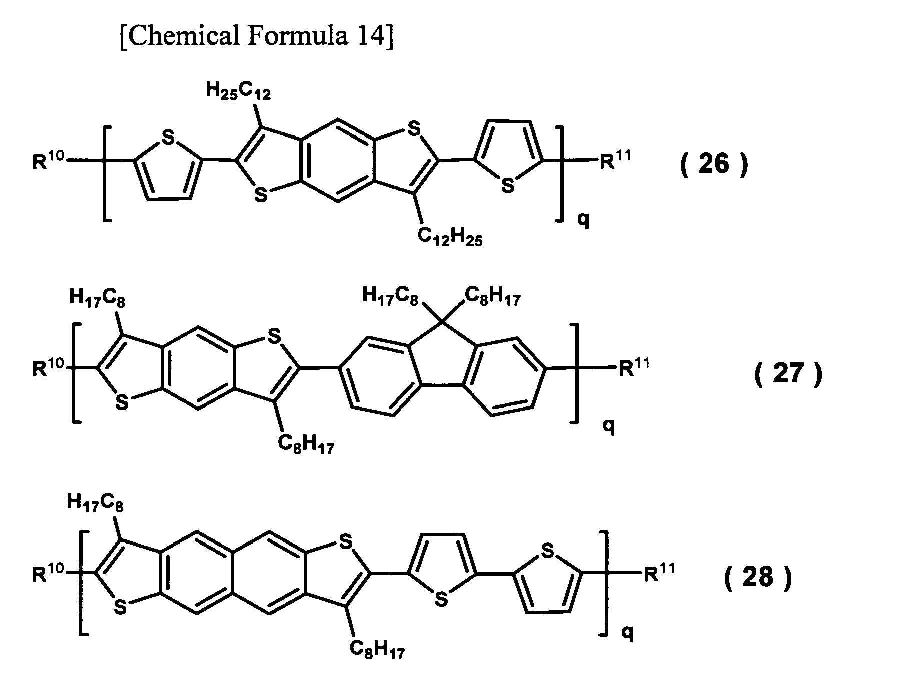

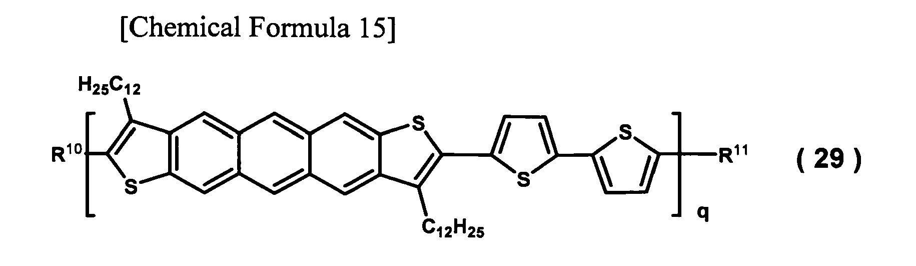

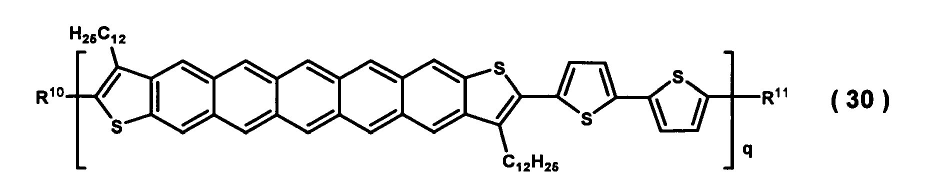

In the above formulae (20) to (30), R¹⁰ and R¹¹ each independently represent the above described terminal group; and an alkyl group, an alkoxy group, an aryl group and a monovalent heterocyclic group are preferable. In addition, p represents an integer of 2 or more, and q is an integer which satisfies q = p - 1. p and q can be appropriately selected according to a method for forming an organic film with the use of the polycyclic fusedring polymer. Specifically, if the polycyclic fusedring polymer has sublimability, the organic film can be formed by using a vapor deposition method such as a vacuum vapor deposition method; and accordingly in this case, p and q are preferably 2 to 10 and further preferably are 2 to 5. On the other hand, when the organic film is formed with a method of applying a solution in which the polycyclic fusedring polymer has been dissolved in an organic solvent, p and q are preferably 3 to 500, more preferably are 6 to 300 and further preferably are 20 to 200.

It is preferable if the number average molecular weight of the above described polycyclic fusedring polymer is 1×10³ to 1×10⁸ in terms of polystyrene, it is more preferable if the number average molecular weight is 1×10³ to 1×10⁶, and it is further preferable if the number average molecular weight is 5×10³ to 1×10⁵, from the viewpoint of enhancing the uniformity of the film when the organic film is formed by application.

### [Method for producing polycyclic fusedring polymer]

A method of polymerizing a polycyclic fusedring compound as will be described below is particularly preferable as the method for producing the polycyclic fusedring polymer having the above described structure, because of being simple. In the following examples, a preferable method for producing the polycyclic fusedring polymer will be described which has a structure including a first structural unit represented by the above formula (1) and/or a second structural unit represented by the above formula (2), and a third structural unit represented by the above formula (3).

Specifically, the polycyclic fusedring polymer of the present embodiment can be obtained by reacting the polycyclic fusedring compound for forming the first structural unit and/or the second structural unit with a raw monomer for forming the third structural unit to form the polymer.

The polycyclic fusedring compound represented by the above formula (5) and a polycyclic fusedring compound in which Y⁵ and Y⁶ are a polymerization-active group in the above formula (7) are preferable for a polycyclic fusedring compound for forming the first structural unit. In addition, the polycyclic fusedring compound represented by the above formula (6) or a polycyclic fusedring compound in which Y⁵ and Y⁶ are a polymerization-active group in the above formula (8) is preferable for a polycyclic fusedring compound for forming the second structural unit. Furthermore, the compound represented by the following formula (31) is preferable for a monomer compound for forming the third structural unit. In addition, a plurality of types for these polycyclic fusedring compound and monomer compounds may be used in combination, respectively.

[Chemical Formula 16] **Y3-Ar³-Y⁴** (31)

Here, in the above formulae (5) and (6), any of the same reference characters as described in the above described "polycyclic fusedring polymer" is synonymous with the above description. Y¹ and Y² each independently represent a halogen atom, a carboxyl group, an alkoxy carbonyl group, an alkyl sulfonate group, an aryl sulfonate group, an aryl alkyl sulfonate group, an alkyl stannyl group, an aryl stannyl group, an aryl alkyl stannyl group, a boric ester residue, a sulfonium methyl group, a phosphonium methyl group, a methyl phosphonate group, a monohalogenated methyl group, a boric acid residue, a formyl group and a vinyl group.

In addition, in the above formulae (7) and (8), any of the same reference characters as described in the above described "polycyclic fusedring polymer" is synonymous with the above description. Y⁵ and Y⁶ each independently represent a hydrogen atom, a halogen atom, an alkyl group having 1 to 20 carbon atoms, an aryl group that has 6 to 60 carbon atoms and may have a substituent, a monovalent heterocyclic group that has 4 to 60 carbon atoms and may have a substituent, a carboxyl group, an alkoxycarbonyl group, an alkyl sulfonate group, an aryl sulfonate group, an aryl alkyl sulfonate group, an alkyl stannyl group, an aryl stannyl group, an aryl alkyl stannyl group, a boric ester residue, a sulfonium methyl group, a phosphonium methyl group, a methyl phosphonate group, a monohalogenated methyl group, a boric acid residue, a formyl group, or a vinyl group. In the above description, the alkyl group may be any of straight, branched and cyclic alkyl groups.

Furthermore, in the above formula (31), Y² and Y³ each independently include similar groups to the groups of the above described Y¹ and Y². Still furthermore, in the above formulae (7), (8) and (31), Ar² and Ar³ each independently represent a divalent aromatic hydrocarbon group which may have a substituent or a divalent heterocyclic group which may have a substituent. In the formulae (7) and (8) in particular, it is preferable if at least one of Ar² and Ar³ is a divalent heterocyclic group which may have a substituent. In this case, it becomes possible to obtain an effect of easily forming a π-π stack structure due to an interaction between heteroatoms in respective molecules.

Among them, when the polycyclic fusedring compound or the monomer compound is used as a raw material of the polycyclic fusedring polymer, it is preferable if Y¹, Y², Y³, Y⁴, Y⁵ and Y⁶ are the polymerization-active group as described above, from the viewpoint of enhancing reactivity. It is preferable that the Y¹, Y², Y³, Y⁴, Y⁵ and Y⁶ be a halogen atom, a carboxyl group, an alkoxy carbonyl group, an alkyl sulfonate group, an aryl sulfonate group, an aryl alkyl sulfonate group, an alkyl stannyl group, a boric ester residue or a boric acid residue, among them.

The polycyclic fusedring polymer can be synthesized by repeatedly causing the reaction of making a bond form between the above described polycyclic fusedring compound and the monomer compound, or form between the polycyclic fusedring compounds. The reaction of making the bond form between these compounds includes a Wittig reaction, a Heck reaction, a Horner-Wadsworth-Emmons reaction, a Knoevenagel reaction, a Suzuki coupling reaction, a Grinard reaction, a Stille reaction and a polymerization reaction with the use of an Ni (O) catalyst. In addition to the above reactions, a reaction caused by the decomposition of an intermediate compound having an adequate leaving group also can be applied, and includes a method of synthesizing poly(p-phenylene vinylene) from an intermediate compound having a sulfonium group. The combination of the groups represented by Y¹, Y², Y³, Y⁴, Y⁵ and Y⁶ can be appropriately selected according to a target reaction.

Among the above described methods for synthesizing the polycyclic fusedring polymer, the process of using the Wittig reaction, the process of using the Heck reaction, the process of using the Horner-Wadsworth-Emmons reaction, the process of using the Knoevenagel reaction, the process of using the Suzuki coupling reaction, the process of using the Grignard reaction, the process of using the Stille reaction, or the process of using the Ni (O) catalyst is preferable, because of easily controlling the structure of the polycyclic fusedring polymer. The process of using the Suzuki coupling reaction, the process of using the Grignard reaction, the process of using the Stille reaction or the process of using the Ni (O) catalyst is particularly preferable, because the raw material is easily obtained and the operation of the reaction is simple.

The synthetic reaction of the polycyclic fusedring polymer can be conducted, for instance, by dissolving the polycyclic fusedring compound or the monomer compound which is a raw material into an organic solvent as needed, and then reacting the compound at a temperature equal to or higher than the melting point and equal to or lower than the boiling point of the organic solvent, in the presence of an alkali and/or a suitable catalyst as needed.

In such a reaction, it is generally preferable to use a solvent which has been subjected to sufficient deoxygenation treatment as the organic solvent in order to suppress a side reaction, though the solvent varies depending on a compound or a reaction to be used, and it is also preferable to use a solvent which has been subjected to dehydration treatment (though this shall not be applied to the case of a reaction in a two-phase system containing water as in the Suzuki coupling reaction). In addition, it is preferable to progress the reaction under an inert atmosphere.

A solvent which is used for the reaction includes: a saturated hydrocarbon such as pentane, hexane, heptane, octane and cyclohexane; an unsaturated hydrocarbon such as benzene, toluene, ethylbenzene and xylene; a halogenated saturated hydrocarbon such as carbon tetrachloride, chloroform, dichloromethane, chlorobutane, bromobutane, chloropentane, bromopentane, chlorohexane, bromohexane, chlorocyclohexane and bromocyclohexane; a halogenated unsaturated hydrocarbon such as chlorobenzene, dichlorobenzene and trichlorobenzene; alcohols such as methanol, ethanol, propanol, isopropanol, butanol and t-butyl alcohol; carboxylic acids such as formic acid, acetic acid and propionic acid; ethers such as dimethyl ether, diethyl ether, methyl-t-butyl ether, tetrahydrofuran, tetrahydropyran and dioxane; and an inorganic acid such as hydrochloric acid, hydrobromic acid, hydrofluoric acid, sulfuric acid and nitric acid. The above described solvents may be used solely, or two or more of the solvents may be concomitantly used.

In addition, the synthetic reaction of the polycyclic fusedring polymer can be efficiently caused by appropriately adding the above described alkali and/or the suitable catalyst. The alkali and the catalyst may be selected according to the reaction to be caused. It is particularly preferable that the alkali and/or the catalyst be sufficiently dissolved in the solvent which is used for the reaction.

When an organic film formed of the polycyclic fusedring polymer is used for the organic film device, the purity of the polycyclic fusedring polymer tends to give influence on the characteristics of the device. Therefore, it is preferable to refine the polycyclic fusedring compound or the monomer compound, which is to be subjected to the reaction, with methods of distillation, sublimation refinement, recrystallization and the like, and then to react the refined compound. It is also preferable to subject the polycyclic fusedring polymer obtained after the synthesis to refinement treatment such as re-precipitation refinement and classification by chromatography.

After the reaction, a polycyclic fusedring polymer can be obtained by subjecting a solution obtained after the reaction to normal post-treatment, for instance, of quenching the solution by water, extracting the polycyclic fusedring polymer with an organic solvent and further distilling the solvent off. The isolation and refinement of the polycyclic fusedring polymer can be conducted by a method of fractionation by chromatography, recrystallization or the like.

The polycyclic fusedring compound which is used for producing the above described polycyclic fusedring polymer can be obtained, for instance, by the reaction represented by the following scheme. In the following examples, a process of producing the polycyclic fusedring compound represented by the above formula (5) will be shown. In the following scheme, m and n are synonymous with the above description; X is synonymous with the above described X¹ and X²; Y is synonymous with the above described Y¹ and Y²; and a group containing R that is bonded to a ring is a similar group to the alkyl group represented as the above described R¹ and R². For information, the polycyclic fusedring compound can be preferably obtained with the following method, but may be produced with another method.

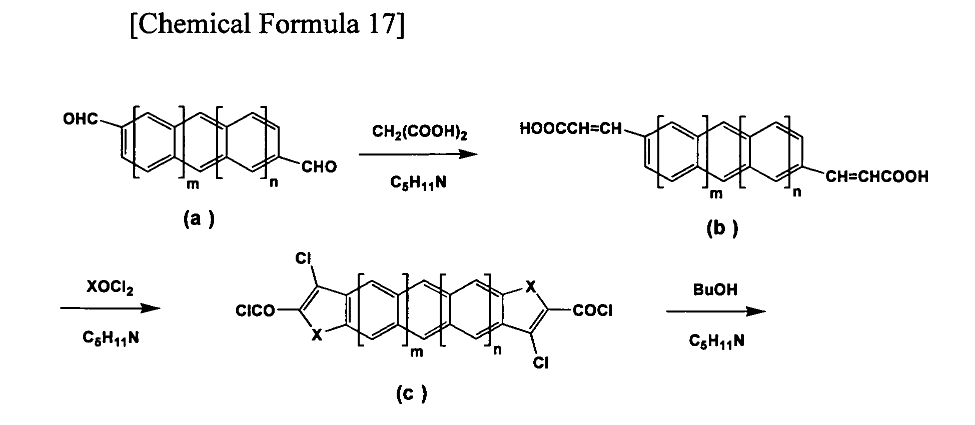

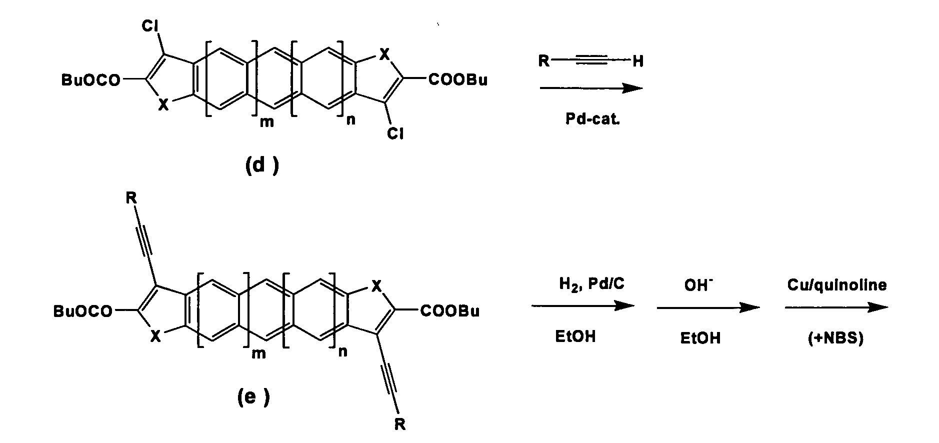

In the reaction of forming such a polycyclic fusedring compound, in order to protect the functional group having high reactivity, a step of converting the functional group to an inactive functional group (protective group) in the following reaction, or a step of removing the protective group after the end of the reaction may be further conducted. Incidentally, the protective group can be appropriately selected according to the functional group to be protected, the reaction to be used and the like, but trimethyl silyl (TMS), triethyl silyl (TES), tert-butyl dimethyl silyl (TBS or TBDMS), triisopropyl silyl (TIPS), tert-butyl phenyl silyl (TBDPS) or the like can be applied for the protection of an active hydrogen, for instance.

In addition, in the reaction of synthesizing the above described polycyclic fusedring compound, a suitable solvent can be used as needed, as is illustrated in the expression. It is preferable that the solvent not obstruct a target reaction if possible. The solvent includes an aliphatic hydrocarbon such as hexane, an aromatic hydrocarbon such as benzene and toluene, a nitrile such as acetonitrile, an ether such as diethyl ether, tetrahydrofuran and 1,2-dimethoxyethane, and a halogenated solvent such as dichloromethane, 1,2-dichloroethane and carbon tetrachloride; and the dichloromethane is preferable. These may be used solely, or two or more of the solvents may be concomitantly used.

For information, as for the above described polycyclic fusedring polymer, and the reaction condition and a reaction reagent to be used in the synthesis with the use of the polycyclic fusedring compound which is a raw material of the polycyclic fusedring polymer, it is possible to appropriately select and use other polymers, reaction conditions and reaction reagents as well than those illustrated in the above description.

In addition, the above described polycyclic fusedring compound also occasionally has high charge transport properties by itself, and accordingly can be occasionally used as a material having charge transport properties as it is. The compounds represented by the above formulae (7) and (8) tend to show particularly high charge transport properties. Among the compounds that are represented by the formula (7) or (8), preferable compounds for the material having charge transport properties include compounds having structures represented by the following formulae (32) to (39). In the following formulae (32) to (39), any of the same reference characters as were described above is synonymous with the above description. R¹² and R¹³ each independently represent a halogen atom, an alkyl group, an alkoxy group, an aryl group or a monovalent heterocyclic group; and among them, the alkyl group is preferable.

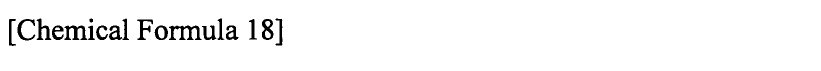

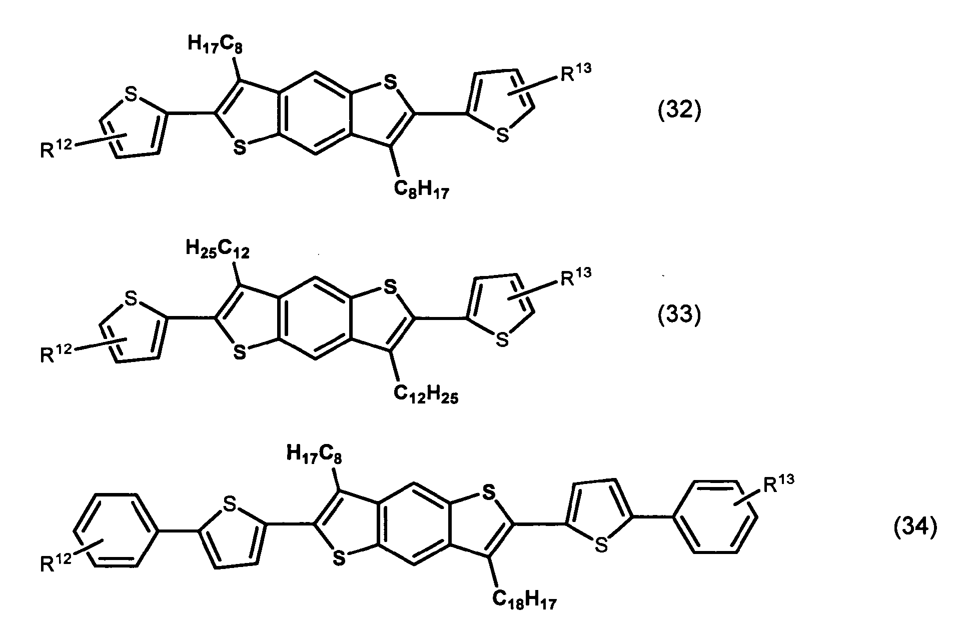

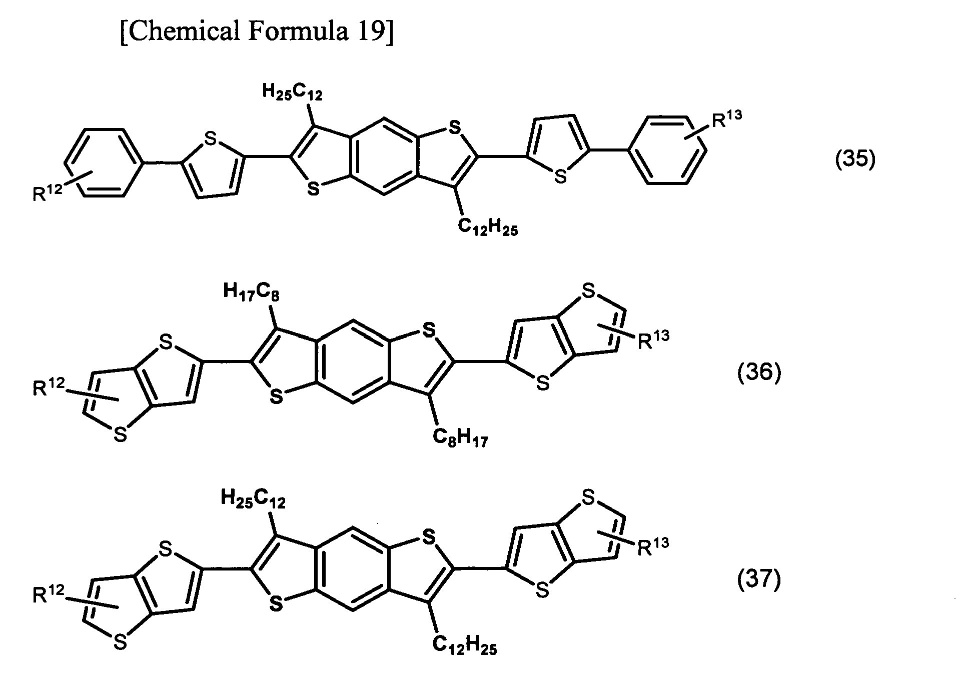

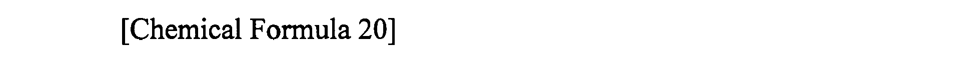

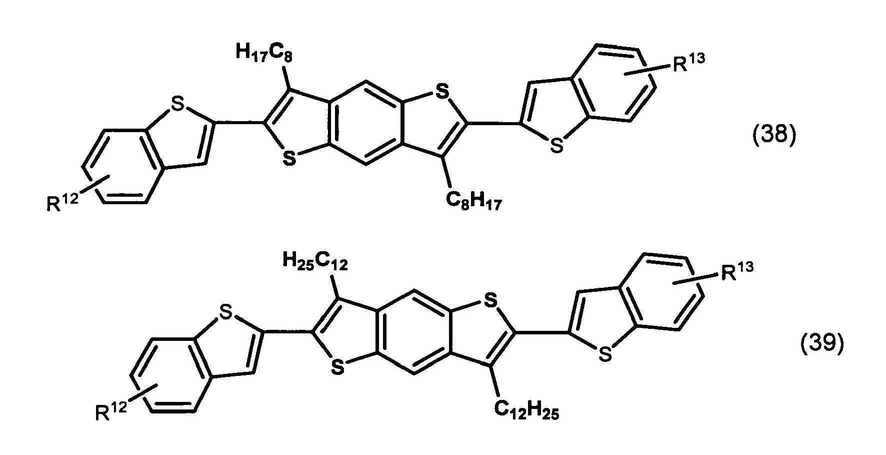

### [Organic film]

Next, an organic film according to preferred embodiments will be described below. The organic film has a structure which contains the polycyclic fusedring polymer or the polycyclic fusedring compound according to the above described embodiment and has a membranal shape.

The preferable thickness of the organic film varies depending on a device to which the organic film is applied, but usually is in a range of 1 nm to 100 µm, is preferably in a range of 2 nm to 1,000 nm, is more preferably in a range of 5 nm to 500 nm, and is further preferably in a range of 20 nm to 200 nm.

The organic film may contain solely one type of the polycyclic fusedring polymer or the polycyclic fusedring compound of the present invention, and may also contain two or more types of the polycyclic fusedring polymer or the polycyclic fusedring compound. In addition, a low-molecular compound or a high-molecular compound other than the polycyclic fusedring polymer or the polycyclic fusedring compound, which has electron transport properties or hole transport properties, can also be used in a mixed form, in order to enhance the electron transport properties or the hole transport properties of the organic film. When the organic film contains a component other than the polycyclic fusedring polymer or the polycyclic fusedring compound, the organic film preferably contains at least 30 mass% or more of the polycyclic fusedring polymer or the polycyclic fusedring compound, and more preferably contains 50 mass% or more. If the content of the polycyclic fusedring polymer or the polycyclic fusedring compound is less than 30 mass%, adequate electric-charge mobility tends to be hardly obtained.

Examples of the compound having the hole transport properties can include a pyrazoline derivative, an arylamine derivative, a stilbene derivative, a triphenyl diamine derivative, an oligothiophene and a derivative thereof, a polyvinyl carbazole and a derivative thereof, polysilane and a derivative thereof, a polysiloxane derivative having an aromatic amine in a side chain or a main chain, polyaniline and a derivative thereof, polythiophene and a derivative thereof, polypyrrole and a derivative thereof, polyphenylene vinylene and a derivative thereof, and polythienylene vinylene and a derivative thereof.

Examples of the compound having the electron transport properties can include an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyl dicyanoethylene and a derivative thereof, a diphenoquinone derivative, 8-hydroxyquinoline and a metal complex of a derivative thereof, polyquinoline and a derivative thereof, polyquinoxaline and a derivative thereof, polyfluorene and a derivative thereof, and fullerenes such as C₆₀ and a derivative thereof.

The organic film may further contain other components so as to enhance its characteristics. The other components include, for instance, a charge-generating material. The organic film contains the charge-generating material, thereby the thin film absorbs a light to generate an electric charge, and the organic film becomes suitable for such use applications like a photosensor as to need to generate the electric charge by absorbing the light.

The charge-generating material includes, for instance, an azo compound and a derivative thereof, a diazo compound and a derivative thereof, a non-metal phthalocyanine compound and a derivative thereof, a metal phthalocyanine compound and a derivative thereof, a perylene compound and a derivative thereof, a polycyclic quinone-based compound and a derivative thereof, a squarylium compound and a derivative thereof, an azulenium compound and a derivative thereof, a thiapyrylium compound and a derivative thereof, and fullerenes such as C₆₀ and a derivative thereof.

In addition, the organic film may further contain the material which is necessary to express various functions. For example, a sensitizer for sensitizing a function of generating the electric charge by adsorbed light, a stabilizer for enhancing stability, a UV absorber for absorbing UV light, and the like are given.

Furthermore, the organic film may further contain a high-molecular material as a polymer binder, from the viewpoint of enhancing its mechanical strength. Such a polymer binder preferably does not excessively lower electron transport properties, and preferably does not excessively absorb a visible light.

The polymer binder includes, for instance, poly(N-vinylcarbazole), polyaniline and a derivative thereof, polythiophene and a derivative thereof, poly(p-phenylene vinylene) and a derivative thereof, poly(2,5-thienylene vinylene) and a derivative thereof, polycarbonate, polyacrylate, polymethyl acrylate, polymethylmethacrylate, polystyrene, polyvinyl chloride and polysiloxane.

The above described organic film can be produced, for instance, by a method as will be described below.

Specifically, the organic film can be formed by applying a solution in which the polycyclic fusedring polymer or the polycyclic fusedring compound and the above described other components as needed have been dissolved in the solvent, onto a predetermined substrate, and removing the solvent by volatilization or the like. However, when the polycyclic fusedring polymer or the polycyclic fusedring compound has sublimability, the organic film can also be formed with a vacuum vapor deposition method or the like.

It is preferable that the solvent can dissolve or uniformly disperse the polycyclic fusedring polymer or the polycyclic fusedring compound and the other components therein. Examples of such a solvent can include: an aromatic hydrocarbon-based solvent such as toluene, xylene, mesitylene, tetralin, decalin and n-butylbenzene; a halogenated saturated hydrocarbon-based solvent such as carbon tetrachloride, chloroform, dichloromethane, dichloroethane, chlorobutane, bromobutane, chloropentane, bromopentane, chlorohexane, bromohexane, chlorocyclohexane and bromocyclohexane; a halogenated aromatic hydrocarbon-based solvent such as chlorobenzene, dichlorobenzene and trichlorobenzene; and an ethers-based solvent such as tetrahydrofuran and tetrahydropyran. It is preferable that 0.1 mass% or more of the polycyclic fusedring polymer or the polycyclic fusedring compound be dissolved in a solvent.

Methods of applying the solution include, for instance, a spin coating method, a casting method, a microgravure coating method, a gravure coating method, a bar coating method, a roll coating method, a wire bar coating method, a dip coating method, a spray coating method, a screen printing method, a flexographic printing method, an offset printing method, an ink jet printing method and a dispenser printing method. Among them, the spin coating method, the flexographic printing method, the ink jet printing method or the dispenser printing method is preferable.

In addition, a step of orienting the polycyclic fusedring polymer or the polycyclic fusedring compound in the organic film may be further conducted for the organic film, according to the use application. By such orientation, the main chain or side chain of the polycyclic fusedring polymer or the polycyclic fusedring compound in the organic film result in being aligned in a fixed direction, and the charge transport properties of the organic film are further enhanced.

A method which is usually used for the orientation of a liquid crystal or the like can be applied to the method of orienting the organic film. Specifically, a rubbing method, a photo-orientation method, a shearing method (shear stress application method), a drawing-up application method and the like are preferable because of being simple and useful, and the rubbing method or the sharing method is more preferable.

### [Organic film device]

The organic film of the above described embodiment results in having excellent charge (electron or hole) transport properties because of containing the polycyclic fusedring polymer or the polycyclic fusedring compound of the above described embodiment. Accordingly, this organic film can efficiently transport electrons or holes which have been injected from an electrode or the like, or electric charges which have been generated due to light absorption or the like, and can be applied to various electric devices (organic film device) with the use of the organic film. In the case that the organic film is used for these organic film devices, it is preferable to orient the organic film by orienting, because higher electron transport property or hole transport property can be obtained. Examples of the organic film device will be each described below.

### (Organic film transistor)

Firstly, the organic film transistor according to preferred embodiments will be described below. The organic film transistor may have a structure having a source electrode and a drain electrode, an organic film layer (active layer) which becomes a current path between the source electrode and the drain electrode and contains the polycyclic fusedring polymer or the polycyclic fusedring compound of the present invention, and a gate electrode which controls current quantity passing through the current path, and the examples include an electric-field effect type and an electrostatic induction type.

The electric-field effect type organic film transistor preferably has a source electrode and a drain electrode, an organic film layer (active layer) which becomes a current path between the source electrode and the drain electrode and contains the polycyclic fusedring polymer or the polycyclic fusedring compound of the present invention, a gate electrode which controls the current quantity passing through the current path, and an insulation layer arranged between the active layer and the gate electrode. It is particularly preferable that the source electrode and the drain electrode are provided in contact with the organic film layer (active layer) containing the polycyclic fusedring polymer or the polycyclic fusedring compound of the present invention, and that the gate electrode is further provided so as to sandwich the insulation layer which comes in contact with the organic film layer.

On the other hand, it is preferable that the electrostatic induction type organic film transistor has a source electrode and a drain electrode, an organic film layer which becomes a current path between the source electrode and the drain electrode and contains the polycyclic fusedring polymer or the polycyclic fusedring compound of the present invention and a gate electrode which controls the current quantity passing through the current path, and that the gate electrode is preferably provided in the organic film layer. It is particularly preferable that the source electrode, the drain electrode and the gate electrode provided in the organic film layer are provided in contact with the organic film layer containing the polycyclic fusedring polymer or the polycyclic fusedring compound of the present invention. The gate electrode may have a structure in which a current path through which an electric current passes from the source electrode to the drain electrode is formed and the quantity of the electric current that passes through the current path can be controlled by voltage applied to the gate electrode, and includes a comb-shaped electrode, for instance.

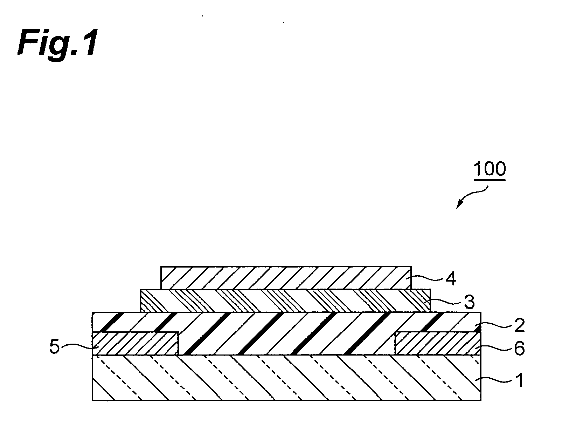

Figure 1 is a schematic sectional view of an organic film transistor (electric-field effect type organic film transistor) according to a first embodiment. The organic film transistor 100 illustrated in Figure 1 includes: a substrate 1; a source electrode 5 and a drain electrode 6 formed on the substrate 1 so as to have a predetermined space; an active layer 2 formed on the substrate 1 so as to cover the source electrode 5 and the drain electrode 6; an insulation layer 3 formed on the active layer 2; and a gate electrode 4 formed on the insulation layer 3 so as to cover a region of the insulation layer 3 between the source electrode 5 and the drain electrode 6.

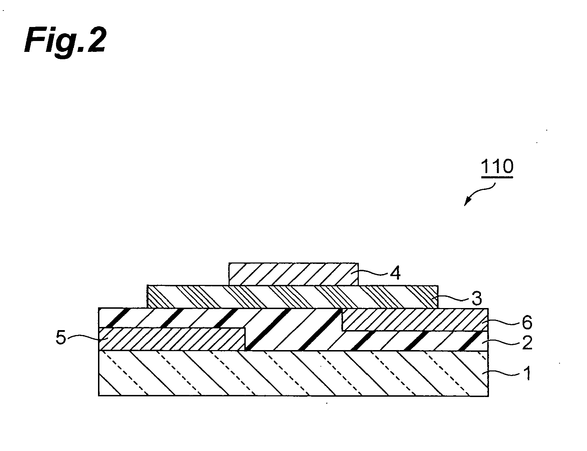

Figure 2 is a schematic sectional view of an organic film transistor (electric-field effect type organic film transistor) according to a second embodiment. The organic film transistor 110 illustrated in Figure 2 includes: a substrate 1; a source electrode 5 formed on the substrate 1; an active layer 2 formed on the substrate 1 so as to cover the source electrode 5; a drain electrode 6 formed on the active layer 2 so as to have a predetermined space to the source electrode 5; an insulation layer 3 formed on the active layer 2 and the drain electrode 6; and a gate electrode 4 formed on the insulation layer 3 so as to cover a region of the insulation layer 3 between the source electrode 5 and the drain electrode 6.

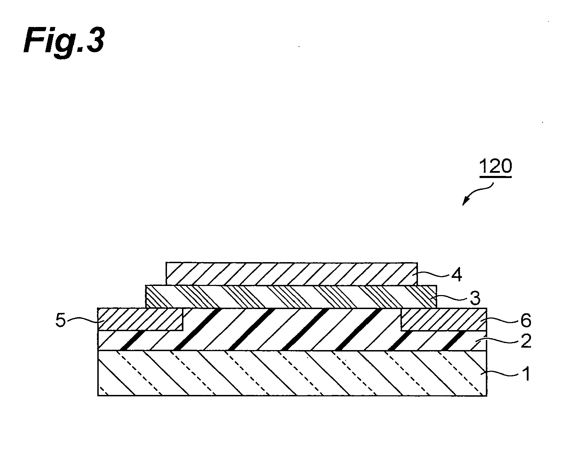

Figure 3 is a schematic sectional view of an organic film transistor (electric-field effect type organic film transistor) according to a third embodiment. The organic film transistor 120 illustrated in Figure 3 includes: a substrate 1; an active layer 2 formed on the substrate 1; a source electrode 5 and a drain electrode 6 formed on the active layer 2 so as to have a predetermined space; an insulation layer 3 formed on the active layer 2 so as to partially cover the source electrode 5 and the drain electrode 6; and a gate electrode 4 formed on the insulation layer 3 so as to partially cover each of a region of the insulation layer 3 having the source electrode 5 formed in its lower part and a region of the insulation layer 3 having the drain electrode 6 formed in its lower part.

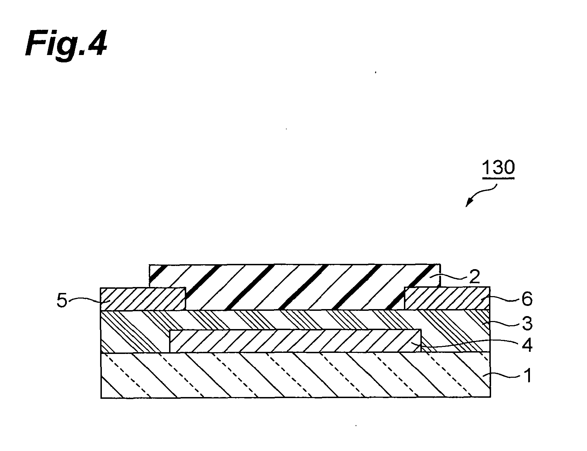

Figure 4 is a schematic sectional view of an organic film transistor (electric-field effect type organic film transistor) according to a fourth embodiment. The organic film transistor 130 illustrated in Figure 4 includes: a substrate 1; a gate electrode 4 formed on the substrate 1; an insulation layer 3 formed on the substrate 1 so as to cover the gate electrode 4; a source electrode 5 and a drain electrode 6 formed on the insulation layer 3 at a predetermined space so as to partially cover a region of the insulation layer 3 having the gate electrode 4 formed in its lower part; and an active layer 2 formed on the insulation layer 3 so as to partially cover the source electrode 5 and the drain electrode 6.

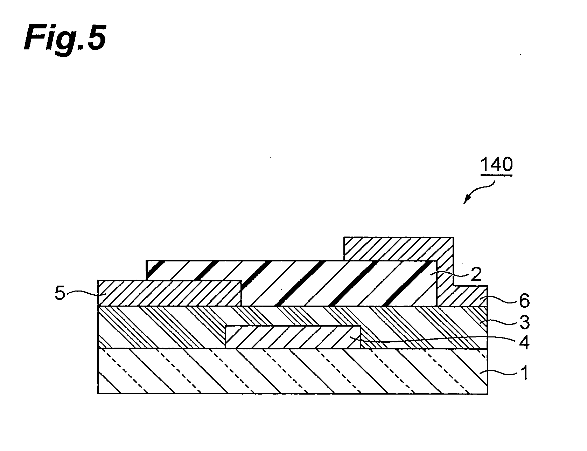

Figure 5 is a schematic sectional view of an organic film transistor (electric-field effect type organic film transistor) according to a fifth embodiment. The organic film transistor 140 illustrated in Figure 5 includes: a substrate 1; a gate electrode 4 formed on the substrate 1; an insulation layer 3 formed on the substrate 1 so as to cover the gate electrode 4; a source electrode 5 formed on the insulation layer 3 so as to partially cover a region of the insulation layer 3 having the gate electrode 4 formed in its lower part; an active layer 2 formed on the insulation layer 3 so as to partially cover the source electrode 5; and a drain electrode 6 formed on the insulation layer 3 so as to partially cover a region of the active layer 2 having the gate electrode 4 formed in its lower part and have a predetermined space to the source electrode 5.

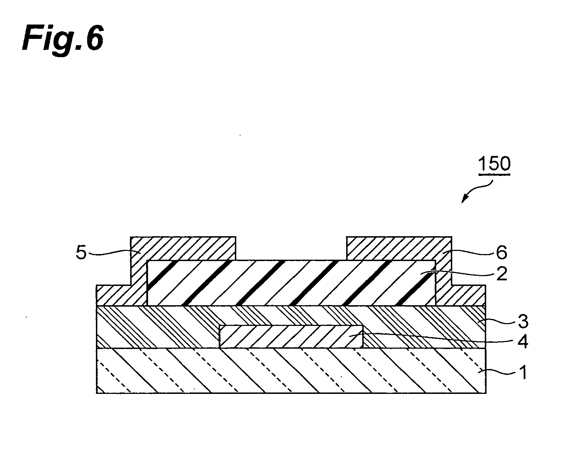

Figure 6 is a schematic sectional view of an organic film transistor (electric-field effect type organic film transistor) according to a sixth embodiment. The organic film transistor 150 illustrated in Figure 6 includes: a substrate 1; a gate electrode 4 formed on the substrate 1; an insulation layer 3 formed on the substrate 1 so as to cover the gate electrode 4; an active layer 2 formed so as to cover a region of the insulation layer 3 having the gate electrode 4 formed in its lower part; a source electrode 5 formed on the insulation layer 3 so as to partially cover a region of the active layer 2 having the gate electrode 4 formed in its lower part; and a drain electrode 6 formed on the insulation layer 3 so as to have a predetermined space to the source electrode 5 and partially cover a region of the active layer 2 having the gate electrode 4 formed in its lower part.

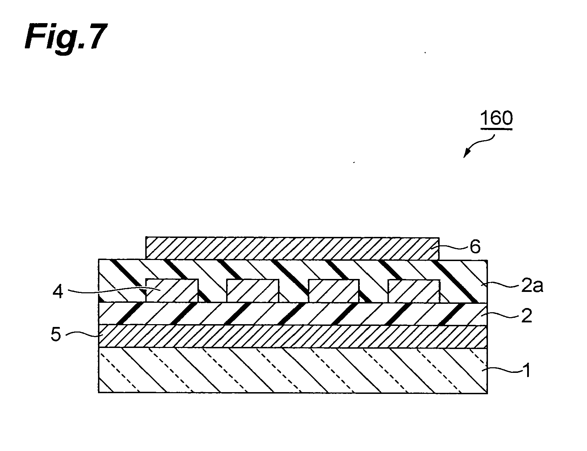

Figure 7 is a schematic sectional view of an organic film transistor (electrostatic induction type organic film transistor) according to a seventh embodiment. The organic film transistor 160 illustrated in Figure 7 includes: a substrate 1; a source electrode 5 formed on the substrate 1; an active layer 2 formed on the source electrode 5; a plurality of gate electrodes 4 formed on the active layer 2 so as to have a predetermined space; an active layer 2a (where the material constituting the active layer 2a may be the same as or different from that of the active layer 2) formed on the active layer 2 so as to cover the whole of the gate electrode 4; and a drain electrode 6 formed on the active layer 2a.

In the organic film transistor according to the first to the seventh embodiments, the active layer 2 and/or the active layer 2a contain the polycyclic fusedring polymer or the polycyclic fusedring compound of the present invention, and become a current path (channel) between the source electrode 5 and the drain electrode 6. The gate electrode 4 controls the current quantity passing through the current path (channel) in the active layer 2 and/or the active layer 2a by the application of voltage.

Such an electric-field effect type organic film transistor can be produced with a well-known method, for instance, a method described in Japanese Patent Application Laid-Open Publication No. 5-110069. An electrostatic induction type organic film transistor can be produced with a well-known method, for instance, a method described in Japanese Patent Application Laid-Open Publication No. 2004-006476.

The substrate 1 is not particularly limited as long as the substrate does not obstruct characteristics of the organic film transistor, but can employ a glass substrate, a flexible film substrate and a plastic substrate.

It is extremely advantageous and preferable in production to use an organic-solvent-soluble compound when forming the active layer 2, and accordingly, the organic film to be active layer 2 can be formed by using a method described above for producing the organic film of the present invention.

A material of the insulation layer 3 contacting the active layer 2 is not particularly limited as long as the material has high electric insulation properties, and can employ a well-known material. The material of the insulation layer 3 includes, for instance, SiOx, SiNx, Ta₂O₅, polyimide, polyvinyl alcohol, polyvinyl phenol, organic glass and a photoresist. A material having a high dielectric constant is more preferable from the viewpoint of lowering voltage.

When forming the active layer 2 on the insulation layer 3, it is also possible to modify the surface of the insulation layer 3 by treating the surface the insulation layer 3 with a surface treatment agent such as a silane coupling agent and then form the active layer 2, in order to enhance interfacial characteristics between the insulation layer 3 and the active layer 2. The surface treatment agent includes, for instance, long-chain alkyl chlorosilanes, long-chain alkyl alkoxy silanes, fluorination alkyl chlorosilanes, fluorinated alkyl alkoxy silanes, and a silyl amine compound such as hexamethyldisilazane. It is also possible to treat the surface of the insulation layer with ozone UV or O₂ plasma before treating the surface with the surface treatment agent.

In addition, it is preferable to form a protective film on the organic film transistor in order to protect the device after having produced the organic film transistor. Thereby, the organic film transistor is blocked from the atmosphere, which can suppress the degradation of the characteristics of the organic film transistor. In addition, the protective film can decrease the influence to be exerted from the step of forming the display device to be driven on the organic film transistor.

The method for forming the protective film includes, for instance, a method of covering the device with a UV curable resin, a thermoset resin or an inorganic SiONₓ film. It is preferable for effectively blocking the device from the atmosphere to conduct a step until forming the protective film after having produced the organic film transistor, without exposing the device to the atmosphere (in dried nitrogen atmosphere or in the vacuum, for instance).

### (Solar cell)

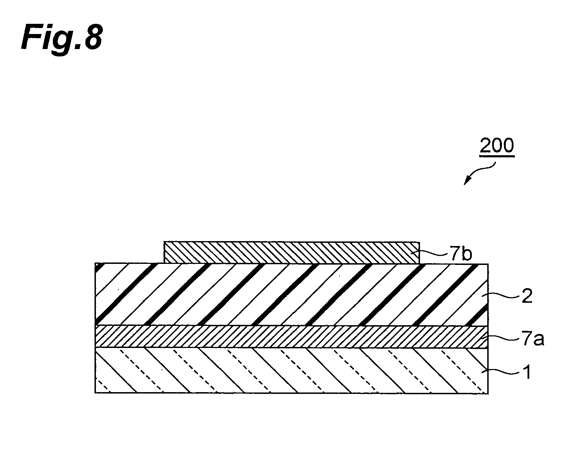

Next, the application of the organic film of the present invention to a solar cell will be described below. Figure 8 is a schematic sectional view of the solar cell according to an embodiment. The solar cell 200 illustrated in Figure 8 includes: a substrate 1; a first electrode 7a formed on the substrate 1; an active layer 2 which is formed of an organic film containing the polycyclic fusedring polymer or the polycyclic fusedring compound of the present invention is formed on the first electrode 7a; and a second electrode 7b formed on the active layer 2.

The solar cell according to the present embodiment uses a transparent or semi-transparent electrode in one of the first electrode 7a and the second electrode 7b. The electrode material can use a metal such as aluminum, gold, silver, copper, an alkaline metal and an alkaline-earth metal, or a semi-transparent film and a transparent electroconductive film thereof. In order to obtain a high open voltage, it is preferable that each of the electrodes is selected so as to have a large difference between the work functions. The active layer 2 (organic film) can employ a charge-generating agent, a sensitizer and the like which are added, in order to enhance the light sensitivity. For a substrate 1, a silicon substrate, a glass substrate, a plastic substrate or the like can be used.

### (Photosensor)

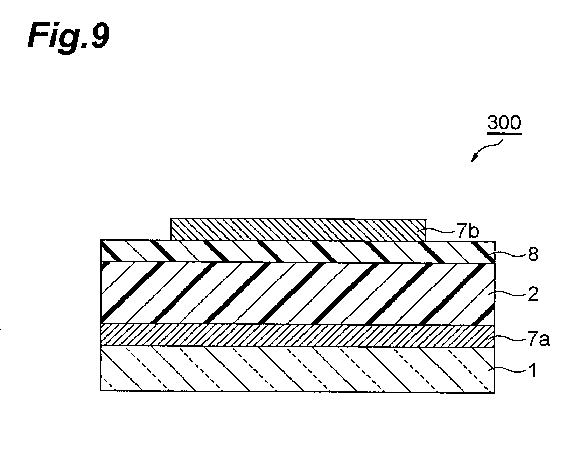

Next, the application of the organic film of the present invention to a photosensor will be described below. Figure 9 is a schematic sectional view of a photosensor according to a first embodiment. The photosensor 300 illustrated in Figure 9 includes: a substrate 1; a first electrode 7a formed on the substrate 1; an active layer 2 which is formed of an organic film containing the polycyclic fusedring polymer or the polycyclic fusedring compound of the present invention and is formed on the first electrode 7a; a charge-generating layer 8 formed on the active layer 2; and a second electrode 7b formed on the charge-generating layer 8.

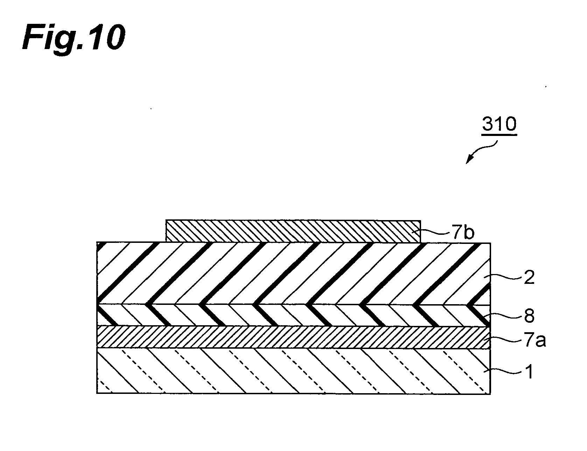

Figure 10 is a schematic sectional view of a photosensor according to a second embodiment. The photosensor 310 illustrated in Figure 10 includes: a substrate 1; a first electrode 7a formed on the substrate 1; a charge-generating layer 8 formed on the first electrode 7a; an active layer 2 which is formed of an organic film containing the polycyclic fusedring polymer or the polycyclic fusedring compound of the present invention and is formed on the charge-generating layer 8; and a second electrode 7b formed on the active layer 2.

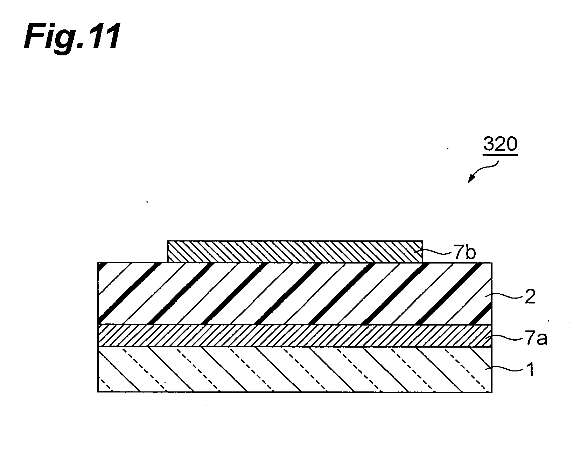

Figure 11 is a schematic sectional view of a photosensor according to a third embodiment. The photosensor 320 illustrated in Figure 11 includes: a substrate 1; a first electrode 7a formed on the substrate 1; an active layer 2 which is formed of an organic film containing the polycyclic fusedring polymer or the polycyclic fusedring compound of the present invention and is formed on the first electrode 7a; and a second electrode 7b formed on the active layer 2.

In the photosensor according to the first to third embodiments, a transparent or semi-transparent electrode is used for one of the first electrode 7a and the second electrode 7b. The charge-generating layer 8 is a layer which absorbs light to generate an electric charge. The electrode material can use a metal such as aluminum, gold, silver, copper, an alkaline metal and an alkaline-earth metal, or a semi-transparent film and a transparent electroconductive film thereof. The active layer 2 (organic film) can employ a carrier-generating agent, a sensitizer and the like which are added, in order to enhance the light sensitivity. For a substrate 1, a silicon substrate, a glass substrate, a plastic substrate or the like can be used.

In the above, the present invention has been described in detail with reference to the embodiments, but the present invention is not limited to the above described embodiments, and can be modified in various ways, in such a range as not to apart from the scope of the present invention. For instance, the organic film device is not limited to the above described embodiment as long as the organic film device is an electric device to which the organic film is applied. The organic film device other than the above description includes, for instance, an organic EL device, an organic memory, a photorefractive device, a spatial light modulator and an imaging device.

### Examples

The present invention will be described further in detail below with reference to examples, but the present invention is not limited to these examples.

### (Measurement condition)

In the following synthesis examples and examples, various analyses or the like were conducted on the following conditions. Firstly, a nuclear magnetic resonance (NMR) spectrum was measured with JNM-GSX-400 made by JEOL Ltd. A gas chromatography mass spectrometry (GC-MS) was conducted with an electron bombardment method while using QP-5050 made by Shimadzu Corporation. High-resolution mass spectrometry (HRMS) was conducted with JMS-DX-303 made by JEOL Ltd. As for the gas chromatograph (GC) analysis, GC-8A made by Shimadzu Corporation was used, on which a glass column (inner diameter of 2.6 mm and length of 1.5 m) filled with silicone OV-17 made by GL Sciences, Inc. was mounted. Wakogel C-200 made by Wako Pure Chemical Industries, Ltd. was used for a silica gel in column chromatographic separation.

### [Example 1]

### <Polycyclic fusedring compound A: synthesis of 3,7-dichlorobenzo[1,2-b:4,5-b']dithiophene-2,6-dibutyl dicarboxylate>

Firstly, 3,7-dichlorobenzo[1,2-b:4,5-b']dithiophene-2,6-dicarbonyl chloride as a starting material was synthesized with reference to the description in a reference document (M. Malesevic, G. Karminski-Zamora, M. Bajic, D. W. Boykin, Heterocycles, 1995, 41, 2691-2699). An esterification reaction was conducted by using the synthesized material, and 3,7-dichlorobenzo[1,2-b:4,5-b']dithiophene-2,6-dibutyl dicarboxylate was synthesized. Specifically, firstly, 3,7-dichlorobenzo[1,2-b:4,5-b']dithiophene-2,6-dicarbonyl chloride (16.5 g, 43 mmol), n-butanol (15.5mL, 172 mmol), pyridine (13.9 mL, 172 mmol), and chlorobenzene (60 mL) were charged into a 200 mL eggplant flask, and were stirred at 100°C for 16.5 hours.

The precipitate was removed by suction-filtration from a solution after the reaction, and chlorobenzene was distilled off from the collected filtrate. The remaining solid was washed and dried. Then, the dried solid was recrystallized with toluene and hexane (while at this time, an insoluble substance was removed by filtration in heating). Thereby, 3,7-dichlorobenzo[1,2-b:4,5-b']dithiophene-2,6-dibutyl dicarboxylate was obtained in a state of a yellow white solid (13.5 g, yield of 65%) (polycyclic fusedring compound A). The measurement result of ¹H-NMR for the obtained object was as follows.
¹H-NMR (400 MHz, CDCl₃): 8 7.95 (s, 2H), 4.41 (t, J=6.4 Hz, 4H), 1.84-1.77 (m, 4H), 1.58-1.48 (m, 4H), 1.02 (t, J=7.3 Hz, 6H)

### [Example 2]

### <Polycyclic fusedring compound B: synthesis of 3,7-di(1-octynyl)benzo[1,2-b:4,5-b']dithiophene-2,6-dibutyl dicarboxylate>

Into a 100 mL two-neck flask, 3,7-dichlorobenzo[1,2-b:4,5-b']dithiophene-2,6-dibutyl dicarboxylate (polycyclic fusedring compound A) (689 mg, 1.5 mmol), 1-octyne (668 mg, 6 mmol), PdCl₂ (PhCN)₂ (11.5 mg, 0.03 mmol), dicyclohexyl (2',4',6'-triisopropylbiphenyl-2-yl)phosphine (42.9 mg, 0.09 mmol), cesium carbonate (3.9 g, 12 mmol), and acetonitrile (10 mL) were charged, and were stirred at 100°C for 24 hours.

The solution after the reaction was extracted with diethyl ether and water, the organic layer was dried with sodium sulfate and then was filtrated with a filter paper, and the solvent was distilled off. By dissolving the residue in toluene, and refining the solute with silica gel column chromatography in which hexane containing 5 wt% ethyl acetate is used as a developing solvent, 3,7-di(1-octynyl)benzo[1,2-b:4,5-b']dithiophene-2,6-dibutyl dicarboxylate was obtained in a state of a yellow solid (370 mg, yield of 41%)(polycyclic fusedring compound B). The measurement result of ¹H-NMR for the obtained object was as follows.
¹H-NMR (400 MHz, CDCl₃) δ 7.99 (s, 2H), 4.39 (t, J=6.4 Hz, 4H), 2.63 (t, J=7.2 Hz, 4H), 1.82-1.70 (m, 8H), 1.57-1.48 (m, 8H), 1.39-1.32 (m, 4H), 1.00 (t, J=7.2 Hz, 6H) , 0.95-0.90 (m, 6H)

### [Example 3]

### <Polycyclic fusedring compound C: synthesis of 3,7-dioctylbenzo[1,2-b:4,5-b']dithiophene-2,6-dibutyl dicarboxylate>

Into a 50 mL autoclave container, 10 wt% Pd/C (52.5 mg, 0.025 mmol) was charged, the inside was replaced with hydrogen, the compound was activated at normal pressure for 1 hour, then a solution in which 3,7-di(1-octynyl)benzo[1,2-b:4,5-b']dithiophene-2,6-dibutyl dicarboxylate (polycyclic fusedring compound B) (308 mg, 0.50 mmol) was dissolved in dehydrated ethanol (5 mL) was charged into the autoclave container, and the mixture was stirred under hydrogen of 15 atmospheric pressure at room temperature for 48 hours.

By filtrating the solution after the reaction with cerite to remove Pd/C, and distilling off the solvent, 3,7-dioctylbenzo[1,2-b:4,5-b']dithiophene-2,6-dibutyl dicarboxylate was obtained in a state of a yellow solid (270 mg, yield of 88%) (polycyclic fusedring compound C). The measurement result of ¹H-NMR for the obtained object was as follows.
¹H-NMR (400 MHz, CDCl₃) δ 7.79 (s, 2H), 4.36 (t, J=6.0 Hz, 4H), 3.33 (t, J=7.6 Hz, 4H), 1.82-1.75 (m, 4H), 1.72-1.65 (m, 4H), 1.61-1.24 (m, 24H), 1.00, (t, J=7.6 Hz, 6H), 0.94-0.86 (m, 6H)

### [Example 4]

### <Polycyclic fusedring compound D: synthesis of 3,7-dioctylbenzo[1,2-b:4,5-b']dithiophene-2,6-dicarboxylic acid>

Into a 100 mL eggplant flask, 3,7-dioctylbenzo[1,2-b:4,5-b']dithiophene-2,6-dibutyl dicarboxylate (polycyclic fusedring compound C) (254 mg, 0.41 mmol), KOH (134 mg, 2.4 mmol), water (1 mL) and ethanol (5 mL) were charged, and were stirred at 100°C for 7 hours.

The ethanol was distilled off from the solution after the reaction, dilute hydrochloric acid was added to the solution, the precipitated solid was suction-filtrated, and thereby 3,7-dioctylbenzo[1,2-b:4,5-b']dithiophene-2,6-dicarboxylic acid was obtained as a yellow white solid (210 mg, yield of 100%) (polycyclic fusedring compound D).

### [Example 5]

### <Polycyclic fusedring compound E: synthesis of 3,7-dioctylbenzo[1,2-b:4,5-b']dithiophene>

Into a 100 mL eggplant flask, 3,7-dioctylbenzo[1,2-b:4,5-b']dithiophene-2,6-dicarboxylic acid (polycyclic fusedring compound D) (150 mg, 0.298 mmol), copper powder (40 mg, 0.6 mmol) and quinoline (1.5 mL) were charged, and the mixture was stirred under nitrogen atmosphere at 260°C for 4 hours.

The solution after the reaction was extracted with diethyl ether, dilute hydrochloric acid and water, the organic layer was dried with sodium sulfate, and then the solvent was distilled off. By dissolving the residue in toluene and refining the solute with silica gel column chromatography in which hexane is used as a developing solvent, 3,7-dioctylbenzo[1,2-b:4,5-b']dithiophene was obtained as a yellow white solid (124 mg, yield of 100%) (polycyclic fusedring compound E). The measurement result of ¹H-NMR for the obtained object was as follows.
¹H-NMR (400 MHz, CDCl₃) δ 7.71 (s, 2H), 7.03 (s, 2H), 2.88 (t, J=7.3 Hz, 6H), 1.81-1.73 (m, 4H), 1.47-1.21 (m, 20H), 0.90-0.84 (m, 6H)

### [Example 6]

### <Polycyclic fusedring compound F: synthesis of 2,6-dibromo-3,7-dioctylbenzo[1,2-b:4,5-b']dithiophene>

Into a 100 mL eggplant flask, 3,7-octylbenzo[1,2-b:4,5-b']dithiophene (polycyclic fusedring compound E) (110 mg, 0.27 mmol), NB S (141 mg, 0.8 mmol) and DMF (3 mL) were charged, and were stirred at room temperature for 4 hours.

The solution after the reaction was extracted with diethyl ether and water, the organic layer was dried with sodium sulfate, and then the solvent was distilled off. By dissolving the residue in toluene and refining the solute with silica gel column chromatography in which hexane is used as a developing solvent, 2,6-dibromo-3,7-octylbenzo[1,2-b:4,5-b']dithiophene was obtained in a state of a yellow white solid (90 mg, yield of 60%) (polycyclic fusedring compound F). The measurement result of ¹H-NMR for the obtained object was as follows.
¹H-NMR (400 MHz, CDCl₃) δ 7.55 (s, 2H), 2.84 (t, J=8.0 Hz, 4H), 1.67-1.59 (m, 4H), 1.45-1.20 (m, 20H), 0.87 (t, J=6.8 Hz, 6H)

### [Example 7]

### <Polycyclic fusedring compound G: synthesis of 3,7-di(1-dodecynyl)benzo[1,2-b:4,5-b']dithiophene-2,6-dibutyl dicarboxylate>

Into a 30 mL two-neck flask, 3,7-dichlorobenzo[1,2-b:4,5-b']dithiophene-2,6-dibutyl dicarboxylate (polycyclic fusedring compound A) (990 mg, 2.15 mmol), 1-dodecyne (1.48 g, 8.9 mmol), PdCl₂ (PhCN)₂ (17 mg, 0.044 mmol), dicyclohexyl (2',4',6'-triisopropyl biphenyl-2-yl)phosphine (65.2 mg, 0.13 mmol), cesium carbonate (5.79 g, 17.8 mmol) and acetonitrile (15 mL) were charged, and were stirred at 100°C for 24 hours.

The solution after the reaction was extracted with diethyl ether and water, the organic layer was dried with sodium sulfate and then was filtrated with a filter paper, and the solvent was distilled off. By dissolving the residue in toluene and refining the solute with silica gel column chromatography in which hexane containing 5 wt% ethyl acetate is used as a developing solvent, 3,7-di(1-dodecynyl)benzo[1,2-b:4,5-b']dithiophene-2,6-dibutyl dicarboxylate was obtained in a state of a yellow white solid (760 mg, yield of 52%) (polycyclic fusedring compound G). The measurement result of ¹H-NMR for the obtained object was as follows.
¹H-NMR (400 MHz, CDCl₃) δ 7.99 (s, 2H), 4.39 (t, J=6.4 Hz, 4H), 2.62 (t, J=6.8 Hz, 4H), 1.83-1.71 (m, 8H), 1.58-1.48 (m, 8H), 1.43-1.18 (m, 24H), 1.00 (t, J=7.6 Hz, 6H), 0.88 (t, J=6.0 Hz, 6H)

### [Example 8]

### <Polycyclic fusedring compound H: synthesis of 3,7-didodecylbenzo[1,2-b:4,5-b']dithiophene-2,6-dibutyl dicarboxylate>

Into a 50 mL autoclave container, 10 wt% Pd/C (128 mg, 0.061 mmol) was charged, the inside was replaced with hydrogen, the compound was activated at normal pressure for 1 hour, then a solution in which 3,7-di(1-dodecynyl)benzo[1,2-b:4,5-b']dithiophene-2,6-dibutyl dicarboxylate (polycyclic fusedring compound G) (742 mg, 1.03 mmol) was dissolved in dehydrated ethanol (7 mL) and dioxane (6 mL) was charged into the autoclave container, and the mixture was stirred under hydrogen of 14 atmospheric pressure at room temperature for 48 hours.

By filtrating the solution after the reaction with cerite to remove Pd/C and distilling off the solvent, 3,7-didodecylbenzo[1,2-b:4,5-b']dithiophene-2,6-dibutyl dicarboxylate was obtained in a state of a yellow white solid (720 mg, yield of 96%) (polycyclic fusedring compound H). The measurement result of ¹H-NMR for the obtained object was as follows.
¹H-NMR (400 MHz, CDCl₃) δ 7.78 (s, 2H), 4.36 (t, J=7.2 Hz, 4H), 3.32 (t, J=7.6, 4H), 1.82-1.75 (m, 4H), 1.72-1.64 (m, 4H), 1.58-1.42 (m, 10H), 1.37-1.13 (m, 30H), 1.01 (t, J=7.6 Hz, 6H), 0.88 (t, J=6.4 Hz, 6H)

### [Example 9]

### <Polycyclic fusedring compound I: synthesis of 3,7-didodecylbenzo[1,2-b:4,5-b']dithiophene-2,6-dicarboxylic acid>

Into a 100 mL eggplant flask, 3,7-didodecylbenzo[1,2-b:4,5-b']dithiophene-2,6-dibutyl dicarboxylate (polycyclic fusedring compound H) (710 mg, 0.97 mmol), KOH (327.2 mg, 5.84 mmol), water (1 mL) and ethanol (12.5 mL) were charged, and were stirred at 100°C for 8 hours.

The ethanol was distilled off from the solution after the reaction, dilute hydrochloric acid was added to the solution, the precipitated solid was suction-filtrated, and thereby 3,7-didodecylbenzo[1,2-b:4,5-b']dithiophene-2,6-dicarboxylic acid was obtained as a yellow white solid (553 mg, yield of 92%) (polycyclic fusedring compound I).

### [Example 10]

### <Polycyclic fusedring compound J: synthesis of 3,7-didodecylbenzo[1,2-b:4,5-b']dithiophene>

Into a 100 mL eggplant flask, 3,7-didodecylbenzo[1,2-b:4,5-b']dithiophene-2,6-dicarboxylic acid (polycyclic fusedring compound I) (553 mg, 0.897 mmol), copper powder (114 mg, 1.74 mmol) and quinoline (4.0 mL) were charged, and were stirred under nitrogen atmosphere at 260°C for 4 hours.

The solution after the reaction was extracted with diethyl ether, dilute hydrochloric acid and water, the organic layer was dried with sodium sulfate, and the solvent was distilled off. By dissolving the residue in toluene and refining the solute with silica gel column chromatography in which hexane is used as a developing solvent, 3,7-didodecylbenzo[1,2-b:4,5-b']dithiophene was obtained as a yellow white solid (320 mg, yield of 69%) (polycyclic fusedring compound J). The measurement result of ¹H-NMR for the obtained object was as follows.
¹H-NMR (400 MHz, CDCl₃) δ 7.70 (s, 2H), 7.02 (s, 2H), 2.88 (t, J=7.6 Hz, 4H), 1.79-1.73 (m, 4H), 1.70-1.57 (m, 4H), 1.48-1.20 (m, 36H), 0.88 (t, J=7.3 Hz, 6H)

### [Example 11]

### <Polycyclic fusedring compound K: synthesis of 2,6-dibromo-3,7-didodecylbenzo[1,2-b:4,5-b']dithiophene>

Into a 100 mL eggplant flask, 3,7-didodecylbenzo[1,2-b:4,5-b']dithiophene (polycyclic fusedring compound J) (298 mg, 0.58 mmol), NBS (307 mg, 1.73 mmol) and DMF (5 mL) were charged, and were stirred at room temperature for 5 hours.

The solution after the reaction was extracted with diethyl ether and water, the organic layer was dried with sodium sulfate, and then the solvent was distilled off. By dissolving the obtained residue in toluene and refining the solute with silica gel column chromatography in which hexane is used as a developing solvent, 2,6-dibromo-3,7-didodecylbenzo[1,2-b:4,5-b']dithiophene was obtained in a state of a yellow white solid (360 mg, yield of 93%) (polycyclic fusedring compound K). The measurement result of ¹H-NMR for the obtained object was as follows.
¹H-NMR (400 MHz, CDCl₃) δ 7.59 (s, 2H), 2.86 (t, J=7.6 Hz, 2H), 1.68-1.61 (m, 4H), 1.45-1.12 (m, 36H), 0.88 (t, J=7.2 Hz, 4H)

### [Example 12]

### <Polycyclic fusedring compound L: synthesis of 2,6-di(2-thienyl)-3,7-didodecylbenzo[1,2-b:4,5-b']dithiophene>

Into a 20 mL two-neck flask, 2,6-dibromo-3,7-didodecylbenzo[1,2-b:4,5-b']dithiophene (polycyclic fusedring compound K) (342 mg, 0.51 mmol), tributyl(2-thienyl)tin (373 mg, 1.02 mmol), tetrakis(triphenylphosphine)palladium (29 mg, 0.025 mmol), DMF (3 mL) and toluene (3 mL) were charged, and were stirred under nitrogen at 85°C for 24 hours.

By distilling off DMF and toluene from the solution after the reaction under a reduced pressure, dissolving the residue in toluene, and refining the solute with silica gel column chromatography in which hexane is used as a developing solvent, 2,6-di(2-thienyl)-3,7-didodecylbenzo[1,2-b:4,5-b']dithiophene was obtained in a state of a yellow white solid (274 mg, yield of 78%) (polycyclic fusedring compound L). The measurement result of ¹H-NMR for the obtained object was as follows.
¹H-NMR (400 MHz, CDCl₃) δ 7.67 (s, 2H), 7.39 (dd, J=5.2, 1.2 Hz, 2H), 7.28 (dd, J=3.6, 1.2 Hz, 2H), 7.12 (dd, J=5.2, 3.6 Hz, 2H), 3.07-3.03 (m, 4H), 1.78-1.72 (m, 4H), 1.52-1.43 (m, 4H), 1.36- 1.20 (m, 32H), 0.88 (t, J=7.0 Hz, 6H)

### [Example 13]

### <Polycyclic fusedring compound M: synthesis of 2,6-di(5-bromothiophen-2-yl)-3,7-didodecylbenzo[1,2-b:4,5-b']dithioph ene>

Into a 100 mL eggplant flask, 2,6-di(2-thienyl)-3,7-didodecylbenzo[1,2-b:4,5-b']dithiophene (polycyclic fusedring compound L) (274 mg, 0.38 mmol), NBS (210 mg, 1.18 mmol) and DMF (4 mL) were charged, and were stirred at room temperature for 5 hours.

By extracting the solution after the reaction with diethyl ether and water, drying the organic layer with sodium sulfate, and distilling off the solvent, 2,6-di(5-bromothiophen-2-yl)-3,7-didodecylbenzo[1,2-b:4,5-b']dithioph ene was obtained in a state of a yellow white solid (340 mg, yield of 100%) (polycyclic fusedring compound M). The measurement result of ¹H-NMR for the obtained object was as follows.
¹H-NMR (400 MHz, CDCl₃) δ 7.66 (s, 2H), 7.06 (d, J=4.0 Hz, 2H), 7.01 (d, J=4.0 Hz, 2H), 3.03-2.98 (m, 4H), 1.70-1.66 (m, 4H), 1.50-1.42 (m, 4H), 1.39-1.20 (m, 32H), 0.88 (t, J=7.0 Hz, 6H)

### [Example 14]

### <Synthesis of polycyclic fusedring polymer C>

In a 50mL Schlenk tube, 2,6-di(5-bromothiophen-2-yl)-3,7-didodecylbenzo[1,2-b:4,5-b']dithioph ene (polycyclic fusedring compound M) (210 mg, 0.25mmol), Ni(COD)₂ (82.5 mg, 0.30 mmol), bipyridyl (48.4 mg, 0.31 mmol) and toluene (7.5 mL) were charged, and were stirred at 110°C for 21 hours.

The solution after the reaction was poured into methanol, and the precipitated sediment was suction-filtrated. The collected solid was refluxed in heptane, and then the solution was suction-filtrated. Heptane was distilled off, and thereby a red solid (35 mg) was obtained (polycyclic fusedring polymer C represented by the following chemical formula (C)). In addition, the solid was refluxed in chlorobenzene and then filtrated, chlorobenzene was distilled off from the filtrate, and a red solid (7 mg) was obtained.

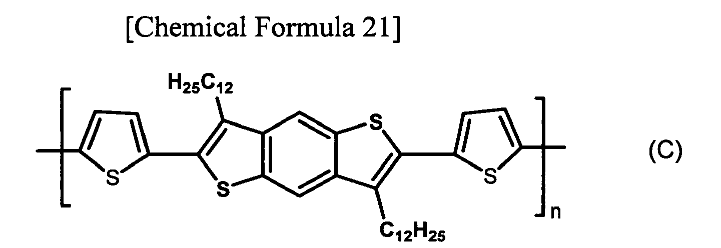

### [Example 15]

### <Production of organic film transistor 1 and evaluation of transistor characteristics thereof>

A substrate is prepared by forming a silicon oxide which will act as an insulation layer by thermal oxidation so as to be 300 nm on the surface of a heavily doped p-type silicon substrate which will act as a gate electrode, and forming a source electrode and a drain electrode further thereon with a lift-off process. An organic film transistor 1 is made by forming an organic film on this substrate with a spin coating method while using a chloroform solution of the polycyclic fusedring polymer C which has been synthesized in Example 14. When the transistor characteristics are measured by applying a gate voltage Vg and a voltage Vsd between the source and the drain to the obtained organic film transistor 1 in the vacuum, adequate drain current - gate voltage (Id-Vg) characteristics are obtained.

### [Example 16]

### <Polycyclic fusedring compound N: synthesis of 2,6-di(2-thienyl)-3,7-dioctylbenzo[1,2-b:4,5-b']dithiophene>

Into a 20 mL two-neck flask, 2,6-dibromo-3,7-dioctylbenzo[1,2-b:4,5-b']dithiophene (polycyclic fusedring compound F) (200 mg, 0.35 mmol), tributyl(2-thienyl)tin (318 mg, 0.84 mmol), tetrakis(triphenylphosphine)palladium (29 mg, 0.025 mmol), DMF (3 mL) and toluene (3 mL) were charged, and were stirred under nitrogen at 85°C for 24 hours.

By distilling off DMF and toluene from the solution after the reaction under a reduced pressure, dissolving the residue in toluene, and refining the solute with silica gel column chromatography in which hexane is used as a developing solvent, 2,6-di(2-thienyl)-3,7-dioctylbenzo[1,2-b:4,5-b']dithiophene was obtained in a state of a yellow white solid (183 mg, yield of 90%) (polycyclic fusedring compound N). The measurement result of ¹H-NMR for the obtained object was as follows.
¹H-NMR (400 MHz, CDCl₃) δ 7.67 (s, 2H), 7.39 (dd, J=5.2, 1.2 Hz, 2H), 7.28 (dd, J=3.6, 1.2 Hz, 2H), 7.12 (dd, J=5.2, 3.6 Hz, 2H), 3.07-3.03 (m, 4H), 1.78-1.69 (m, 4H), 1.52-1.43 (m, 4H), 1.36- 1.20 (m, 16H), 0.88 (t, J=7.0 Hz, 6H)

### [Example 17]

### <Polycyclic fusedring compound O: synthesis of 2,6-di(5-bromothiophen-2-yl)-3,7-dioctylbenzo[1,2-b:4,5-b']dithiophene >

Into a 100 mL eggplant flask, 2,6-di(2-thienyl)-3,7-dioctylbenzo[1,2-b:4,5-b']dithiophene (polycyclic fusedring compound N) (143 mg, 0.25 mmol), NBS (115 mg, 0.65 mmol) and DMF (3 mL) were charged, and were stirred at room temperature for 5 hours.

By extracting the solution after the reaction with diethyl ether and water, drying the organic layer with sodium sulfate, and distilling off the solvent, 2,6-di(5-bromothiophen-2-yl)-3,7-dioctylbenzo[1,2-b:4,5-b']dithiophene was obtained in a state of a yellow white solid (138 mg, yield of 75%) (polycyclic fusedring compound O). The measurement result of ¹H-NMR for the obtained object was as follows.
¹H-NMR (400 MHz, CDCl₃) δ 7.66 (s, 2H), 7.06 (d, J=4.0 Hz, 2H), 7.01 (d, J=4.0 Hz, 2H), 3.03-2.98 (m, 4H), 1.73-1.66 (m, 4H), 1.50-1.40 (m, 4H), 1.40-1.20 (m, 16H), 0.89 (t, J=7.0 Hz, 6H)

### [Example 18]

### <Polycyclic fusedring compound P: synthesis of 2,6-di(5-phenylthiophen-2-yl)-3,7-dioctylbenzo[1,2-b:4,5-b']dithiophen e>

Into a 20 mL reactor, 2,6-di(5-bromothiophen-2-yl)-3,7-dioctylbenzo[1,2-b:4,5-b']dithiophene (polycyclic fusedring compound O) (130 mg, 0.18 mmol), dihydroxyphenylborane (65.8 mg, 0.54 mmol), palladium acetate (2.0 mg, 0.009 mmol), 2-(di-t-butylphosphino)biphenyl (5.4 mg, 0.018 mmol), potassium fluoride (48.8 mg, 0.84 mmol) and toluene (3 mL) were charged, and were stirred at 100°C for 10 hours.

By extracting the solution after the reaction with diethyl ether and water, drying the organic layer with sodium sulfate, distilling off the solvent, dissolving the residue in toluene, refining the solute with silica gel column chromatography in which toluene is used as a developing solvent, and then cleaning the solute with acetone, 2,6-di(5-phenylthiophen-2-yl)-3,7-dioctylbenzo[1,2-b:4,5-b']dithiophen e was obtained in a state of a yellow solid (100 mg, yield of 76%) (polycyclic fusedring compound P). The measurement result of ¹H-NMR for the obtained object was as follows.
¹H-NMR (400 MHz, CDCl₃) δ 7.68 (s, 2H), 7.65 (d, J=4.0, 4H), 7.41 (t, J=7.7 Hz, 4H), 7.32 (dd, J=3.6 Hz, 4H), 7.26 (d, J=4.0 Hz, 4H), 3.03-2.98 (m, 4H), 1.73-1.66 (m, 4H), 1.50- 1.40 (m, 4H), 1.40-1.20 (m, 16H), 0.89 (t, J=7.0 Hz, 6H)

### [Example 19]

### <Polycyclic fusedring compound Q: synthesis of 2,6-di(5-phenylthiophen-2-yl)-3,7-didodecylbenzo[1,2-b:4,5-b']dithioph ene>

Into a 20 mL reactor, 2,6-di(5-bromothiophen-2-yl)-3,7-didodecyl benzo[1,2-b:4,5-b']dithiophene (polycyclic fusedring compound M) (300 mg, 0.35 mmol), dihydroxyphenylborane (128 mg, 1.05 mmol), palladium acetate (3.9 mg, 0.018 mmol), 2-(di-t-butylphosphino)biphenyl (10.4 mg, 0.035 mmol), potassium fluoride (162.6 mg, 2.8 mmol) and toluene (4 mL) were charged, and were stirred at 100°C for 10 hours.

By extracting the solution after the reaction with diethyl ether and water, drying the organic layer with sodium sulfate, distilling off the solvent, dissolving the residue in toluene, refining the solute with silica gel column chromatography in which toluene is used as a developing solvent, and then cleaning the solute with acetone, 2,6-di(5-phenylthiophen-2-yl)-3,7-didodecylbenzo[1,2-b:4,5-b']dithioph ene was obtained in a state of a yellow solid (180 mg, yield of 61%) (polycyclic fusedring compound Q). The measurement result of ¹H-NMR for the obtained object was as follows.
¹H-NMR (400 MHz, CDCl₃) δ 7.68 (s, 2H), 7.65 (d, J=4.0, 4H), 7.41 (t, J=7.7 Hz, 4H), 7.32 (dd, J=3.6 Hz, 4H), 7.26 (d, J=4.0 Hz, 4H), 3.03-2.98 (m, 4H), 1.73-1.66 (m, 4H), 1.50- 1.40 (m, 4H), 1.40-1.20 (m, 32H), 0.89 (t, J=7.0 Hz, 6H)

### [Example 20]

### <Polycyclic fusedring compound R: synthesis of 2,6-di(2-benzo[b]thienyl)-3,7-didodecylbenzo[1,2-b:4,5-b']dithiophene>

Into a 20 mL reactor, 2,6-dibromo-3,7-didodecylbenzo[1,2-b:4,5-b']dithiophene (polycyclic fusedring compound K) (240 mg, 0.36 mmol), tributyl(2-benzo[b]thienyl)tin (181 mg, 0.426 mmol), tetrakis(triphenylphosphine)palladium (20.8 mg, 0.018 mmol), DMF (2 mL) and toluene (2 mL) were charged, and were stirred under nitrogen at 85°C for 24 hours.

By distilling off DMF and toluene from the solution after the reaction under a reduced pressure, dissolving the residue in toluene, and refining the solute with silica gel column chromatography in which hexane is used as a developing solvent, 2,6-di(2-benzo[b]thienyl)-3,7-didodecylbenzo[1,2-b:4,5-b']dithiophene was obtained in a state of a yellow white solid (77.5 mg, yield of 27%) (polycyclic fusedring compound R). The measurement result of ¹H-NMR for the obtained object was as follows.
¹H-NMR (400 MHz, CDCl₃) δ 7.85 (d, J=7.6 Hz, 2H), 7.81 (d, J=7.6 Hz, 2H), 7.67 (s, 2H), 7.51 (s, 2H), 7.41- 7.33 (m, 4H), 3.17-3.13 (m, 4H), 1.82-1.72 (m, 4H), 1.68- 1.63 (m, 4H), 1.53-1.46 (m, 4H), 1.40-1.28 (m, 28H), 0.88 (t, J=7.0 Hz, 6H)

### [Example 21]

### <Synthesis of polycyclic fusedring polymer D>

Into a 20 mL reactor, 2,6-dibromo-3,7-didodecylbenzo[1,2-b:4,5-b']dithiophene (polycyclic fusedring compound K) (199 mg, 0.29 mmol), 5,5'-bis(tributylstannyl)-2,2'-bithiophene (214 mg, 0.29 mmol), tetrakis(triphenylphosphine)palladium (17.3 mg, 0.015 mmol) and chlorobenzene (10 mL) were charged, and were stirred under nitrogen at 85°C for 48 hours.

The solution after the reaction was poured into 200 mL of methanol containing 10 mL of concentrated hydrochloric acid, and the precipitated sediment was suction-filtrated. The collected solid was cleaned with acetone and hexane, the solution was suction-filtrated, and thereby a red black solid (135 mg) was obtained (polycyclic fusedring polymer D represented by the following chemical formula (D)). The number average molecular weight of the polycyclic fusedring polymer D in terms of polystyrene was 14,000.

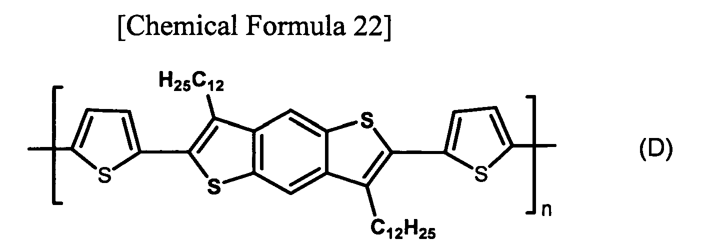

### [Example 22]

### <Synthesis of polycyclic fusedring polymer E>

Into a 50 mL three-neck flask, 5,5'-bis(4,4,5,5-tetramethyl-1,3,5-dioxaborolan-2-yl)-2,2'-bithiophene (319 mg, 0.4 mmol), 2,6-di(5-bromothiophen-2-yl)-3,7-didodecylbenzo[1,2-b:4,5-b']dithioph ene (polycyclic fusedring compound M) (340 mg, 0.4 mmol), tris(dibenzylideneacetone)dipalladium (O) (7.3 mg, 0.008 mmol), tri-tert-butylphosphonium tetrafluoroborate (9.3 mg, 0.032 mmol) and tetrahydrofuran (7.7 mL) were charged. An aqueous solution of potassium carbonate (2 mol/L and 0.6 mL) was added to the mixture, and then the resultant mixture was reacted at 80°C for 5 hours.

Chlorobenzene and water were added to the solution after the reaction, the mixture was stirred at 120°C for 15 minutes, and the water layer was removed. Then, the remaining chlorobenzene solution after the water layer had been removed was concentrated, and then this solution was poured into methanol. Thereby precipitated sediment was filtrated, and a red solid (20 mg) was obtained (polycyclic fusedring polymer E represented by the following chemical formula (E)). The number average molecular weight of the polycyclic fusedring polymer E in terms of polystyrene was 38,000.

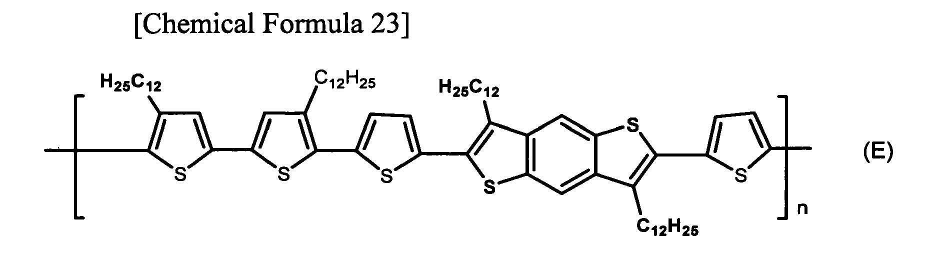

### [Example 23]

### <Production of organic film transistor 2, and evaluation of transistor characteristics thereof>

A silicon oxide which would act as an insulation layer was formed by thermal oxidation so as to be 300 nm on the surface of a heavily doped p-type silicon substrate which would act as a gate electrode, and then this substrate was immersed into an octadecyltrichlorosilane / octane (200 µL / 25 mL) solution in nitrogen for 15 hours, by which the surface of the silicon oxide was modified.

An organic film containing the polycyclic fusedring compound Q was formed by forming an organic film on this silicon oxide with a spin coating method with the use of a 0.5 wt% toluene solution of the polycyclic fusedring compound Q which was synthesized in Example 19, and heating the organic film in nitrogen at 60°C for 30 minutes. An organic film transistor 2 was obtained by forming a source electrode and a drain electrode (channel length/channel width = 20 µm / 2,000 µm) which are formed of MoO₃ (15 nm) / Au (100 nm), on the obtained organic film, with a vacuum vapor-deposition method.

The transistor characteristics were measured by applying a gate voltage Vg of 0 to -60 V and a voltage Vsd of 0 to -60 V between the source and the drain to the obtained organic film transistor 2 in the vacuum, and then adequate drain current - gate voltage (Id-Vg) characteristics were obtained. The mobility at this time was 3×10⁻³ cm²/Vs, the threshold voltage was -36 V, and the ON/OFF ratio was 10⁴. From the result, it was confirmed that the organic film transistor 2 with the use of the polycyclic fusedring compound Q effectively functioned as a p-type organic transistor.

### [Example 24]

### <Production of organic film transistor 3, and evaluation of transistor characteristics thereof>

An organic film transistor 3 was obtained in the same manner as in Example 23, except that the polycyclic fusedring compound P which was synthesized in Example 18 was used in place of the polycyclic fusedring compound Q.

The transistor characteristics were measured by applying a gate voltage Vg of 0 to -60 V and a voltage Vsd of 0 to -60 V between the source and the drain to the obtained organic film transistor 3 in the vacuum, and then adequate drain current - gate voltage (Id-Vg) characteristics were obtained. The mobility at this time was 3×10⁻3 cm²/Vs, the threshold voltage was -36 V, and the ON/OFF ratio was 10⁴. From the result, it was confirmed that the organic film transistor 3 with the use of the polycyclic fusedring compound P effectively functioned as a p-type organic transistor.

## Claims

1. A polycyclic fusedring polymer comprising: two or more first structural units represented by the following formula (1) and/or two or more second structural units represented by the following formula (2); or the first structural unit and the second structural unit in combination: 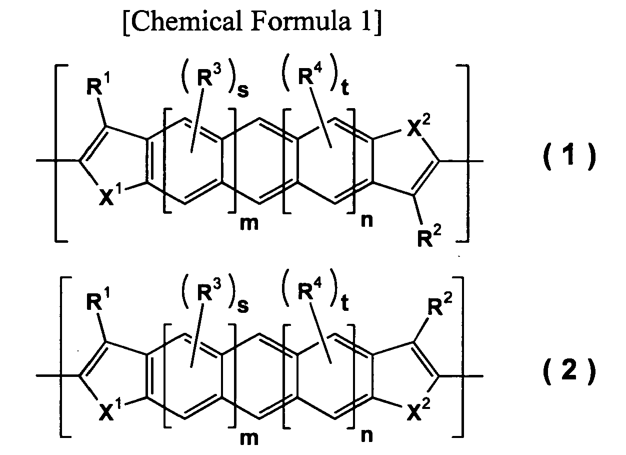 wherein X¹ and X² each independently represent an oxygen atom, a sulfur atom or a selenium atom and R¹ and R² each independently represent a halogen atom, an unsaturated alkyl group, a straight, branched or cyclic alkyl group having 8 to 20 carbon atoms, a non-alkyl group containing a straight, branched or cyclic alkyl group, an aryl group that has 6 to 60 carbon atoms and may have a substituent or a monovalent heterocyclic group that has 4 to 60 carbon atoms and may have a substituent; R³ and R⁴ each independently represent a monovalent group that may have a substituent, and when there are a plurality of R³s and a plurality of R⁴s, the R³s may be the same as or different from each other and the R⁴s may be the same as or different from each other; and m, n, s and t are each independently an integer of 0 to 2.

2. The polycyclic fusedring polymer according to claim 1, wherein the X¹ and the X² are a sulfur atom.

3. The polycyclic fusedring polymer according to claim 1 or 2, further comprising a third structural unit represented by the following formula (3): 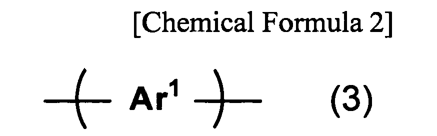 wherein Ar¹ represents a divalent aromatic hydrocarbon group that may have a substituent, or a divalent heterocyclic group that may have a substituent.

4. The polycyclic fusedring polymer according to claim 3, wherein the Ar¹ is a group represented by the following formula (4): 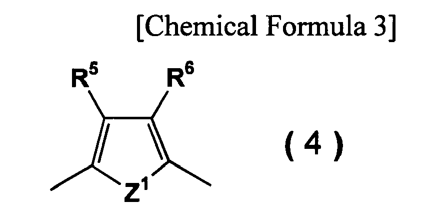 wherein R⁵ and R⁶ each independently represent a hydrogen atom, a halogen atom or a monovalent group and R⁵ and R⁶ may be bonded together to form a ring; and Z¹ represents any one of groups represented by the following formulae (i), (ii), (iii), (iv), (v), (vi), (vii), (viii) and (ix): 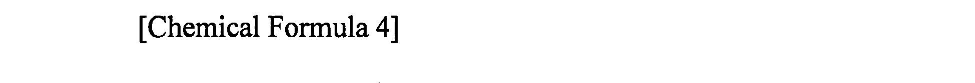 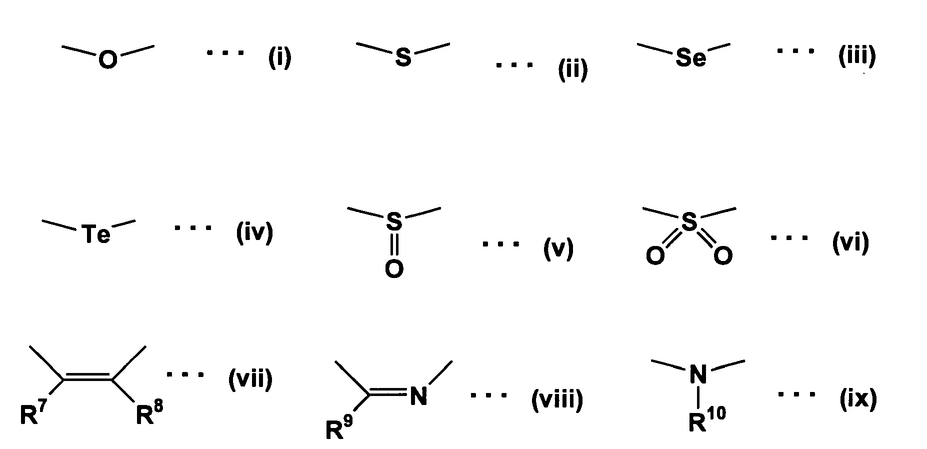 wherein R⁷, R⁸, R⁹ and R¹⁰ each independently represent a hydrogen atom, a halogen atom or a monovalent group, and R⁷ and R⁸ may be bonded together to form a ring; and the left side and right side of the group represented by formula (viii) may be interchanged.

5. The polycyclic fusedring polymer according to claim 4, wherein the Z¹ is a group represented by the formula (ii).

6. A polycyclic fusedring compound represented by the following formula (5) or the following formula (6): 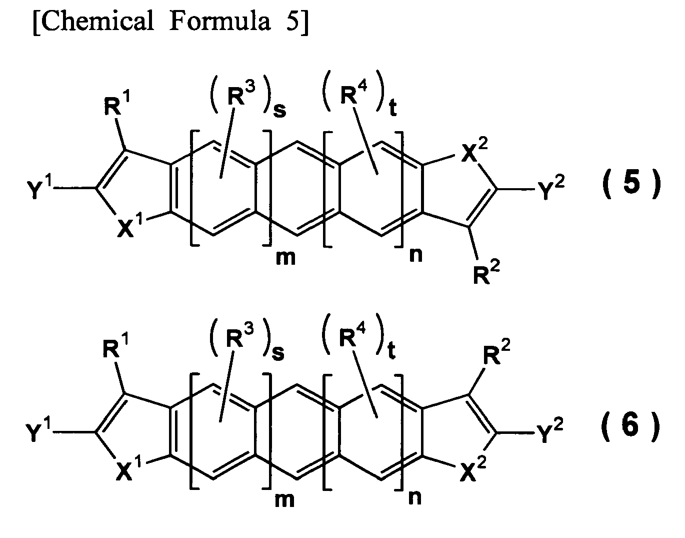 wherein X¹ and X² each independently represent an oxygen atom, a sulfur atom or a selenium atom, R¹ and R² each independently represent a halogen atom, an unsaturated alkyl group, a straight, branched or cyclic alkyl group having 8 to 20 carbon atoms, a non-alkyl group containing a straight, branched or cyclic alkyl group, an aryl group that has 6 to 60 carbon atoms and may have a substituent, or a monovalent heterocyclic group that has 4 to 60 carbon atoms and may have a substituent; R³ and R⁴ each independently represent a monovalent group that may have a substituent, and when there are a plurality of R³s and a plurality of R⁴s, the R³s may be the same as or different from each other and the R⁴s may be the same as or different from each other; Y¹ and Y² each independently represent a halogen atom, a carboxyl group, an alkoxycarbonyl group, an alkyl sulfonate group, an aryl sulfonate group, an aryl alkyl sulfonate group, an alkyl stannyl group, an aryl stannyl group, an aryl alkyl stannyl group, a boric ester residue, a sulfonium methyl group, a phosphonium methyl group, a methyl phosphonate group, a monohalogenated methyl group, a boric acid residue, a formyl group or a vinyl group; and m, n, s and t are each independently an integer of 0 to 2.

7. A polycyclic fusedring compound represented by the following formula (7) or the following formula (8): 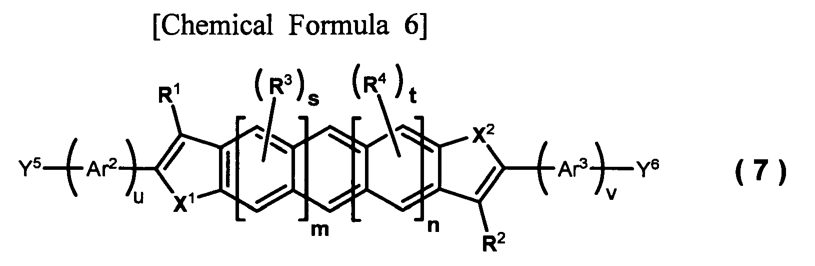 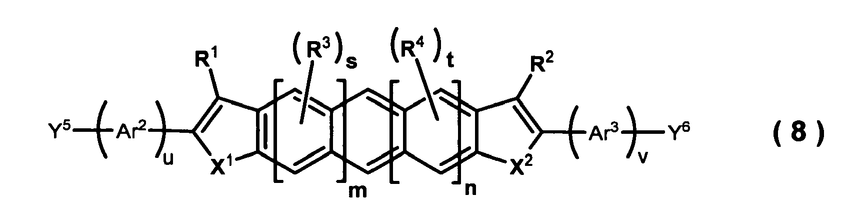 wherein X¹ and X² each independently represent an oxygen atom, a sulfur atom or a selenium atom, R¹ and R² each independently represent a straight, branched or cyclic alkyl group having 8 to 20 carbon atoms, a non-alkyl group containing a straight, branched or cyclic alkyl group, an aryl group that has 6 to 60 carbon atoms and may have a substituent, or a monovalent heterocyclic group that has 4 to 60 carbon atoms and may have a substituent; R³ and R⁴ each independently represent a monovalent group that may have a substituent, and when there are a plurality of R³s and a plurality of R⁴s, the R³s may be the same as or different from each other and the R⁴s may be the same as or different from each other; Y⁵ and Y⁶ each independently represent a hydrogen atom, a halogen atom, an alkyl group having 1 to 20 carbon atoms, an aryl group that has 6 to 60 carbon atoms and may have a substituent, a monovalent heterocyclic group that has 4 to 60 carbon atoms and may have a substituent, a carboxyl group, an alkoxycarbonyl group, an alkyl sulfonate group, an aryl sulfonate group, an aryl alkyl sulfonate group, an alkyl stannyl group, an aryl stannyl group, an aryl alkyl stannyl group, a boric ester residue, a sulfonium methyl group, a phosphonium methyl group, a methyl phosphonate group, a monohalogenated methyl group, a boric acid residue, a formyl group, or a vinyl group; Ar² and Ar³ each independently represent a divalent aromatic hydrocarbon group that may have a substituent, or a divalent heterocyclic group that may have a substituent; and m, n, s and t are each independently an integer of 0 to 2, and u and v are each independently 0 or 1 wherein u + v is a number of 1 or more.

8. The polycyclic fusedring compound according to claim 6 or 7, wherein the X¹ and the X² are a sulfur atom.

9. An organic film comprising the polycyclic fusedring polymer according to any one of claims 1 to 5 or the polycyclic fusedring compound according to claim 7.

10. An organic film device comprising the organic film according to claim 9.

11. An organic film transistor comprising the organic film according to claim 9.
